(19) Europäisches Patentamt
European Patent Office
Office européen des brevets

(11) Publication number: **0 167 548 B1**

(12) **EUROPEAN PATENT SPECIFICATION**

(45) Date of publication of patent specification: **10.02.93**

(51) Int. Cl.⁵: **C12N 15/27**, C07G 17/00, C07H 21/04, C07K 13/00

(21) Application number: **85900031.7**

(22) Date of filing: **24.12.84**

(86) International application number: **PCT/AU84/00272**

(87) International publication number: **WO 85/02863 (04.07.85 85/15)**

(54) **CLONING OF cDNA AND EXPRESSION OF MURINE-INTERLEUKIN-3.**

(30) Priority: **23.12.83 AU 2994/83**

(43) Date of publication of application: **15.01.86 Bulletin 86/03**

(45) Publication of the grant of the patent: **10.02.93 Bulletin 93/06**

(84) Designated Contracting States: **AT BE CH DE FR GB LI LU NL SE**

(56) References cited:
**EP-A- 0 138 133**

**PROC. NATL. ACAD. SCI. USA, vol. 81, February 1984, pages 1070-1074, Washington, US; T. YOKOTA et al.: "Isolation and characterization of a mouse cDNA clone that expresses mast-cell growth-factor activity in monkey cells"**

(73) Proprietor: **THE AUSTRALIAN NATIONAL UNIVERSITY**

**Acton, Australian Capital Territory 2601(AU)**

Proprietor: **BIOTECHNOLOGY AUSTRALIA PTY. LTD.**
**28 Barcoo Street**
**Roseville, NSW 2069(AU)**

(72) Inventor: **YOUNG, Ian, Gordon**
**12 Florina Place**
**Hawker, ACT 2614(AU)**
Inventor: **HAPEL, Andrew, John**
**7 Scoble Place**
**Mawson, ACT 2607(AU)**
Inventor: **JOHNSON, Rosemary, Anne**
**61 Darwinia Terrace**
**Rivett, ACT 2611(AU)**
Inventor: **YMER, Sanie/Burton & Garran Hall**
**Australian National University**
**Acton, ACT 2601(AU)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

**4th International Lymphokine Workshop on Molecular and Cellular Biology of Lymphokines, Oct. 17-21 1984, Lymphokine Res 3(4) 1984, 247, Fung M.C., Hapel A.J. et al. "Biological Properties of Molecularly Cloned and Expressed Murine Interleukin-3"**

**4th International Lymphokine Workshop on Molecular and Cellular Biology of Lymphokines, Oct. 17-21 1984, Lymphokine Res 3(4) 1984, 243, Fung M.C., Hapel A.J. et al. "Cloning and Expression of the Gene for Murine Interleukin-3"**

**Nature (Lond) 307 (5948) 1984, 233-237, Fung M.C., Hapel A.J. et al. "Molecular Cloning of Complement DNA for Murine Interleukin 3"**

**J. Immunol 128(6) 1982, 2393-2398, Lee J.C., Hapel A.J. et al. "Constitutive Production of a Unique Lymphokine Interleukin 3 by the WEH I-3 Cell Line"**

**J. Immunol 132(4) 1984, 1869-1871, Guerne P.A., Piquet P.F. et al. "Production of Interleukin 2 Interleukin 3 and Interferon by Mouse T Lymphocyte Clones of LYT-2-Positive and LYT-2-Negative Phenotype"**

Inventor: **COHEN, Donna, Ruth**
**29 Wentworth Gardens**
**Eyre Street Kingston, ACT 2604(AU)**
Inventor: **CAMPBELL, Hugh, Douglas**
**72 Stuart Street**
**Narrabundah, ACT 2604(AU)**
Inventor: **CLARK, Susan, Joy**
**41 Bellevue Street**
**Chatswood, NSW 2067(AU)**
Inventor: **STIRZACKER, Sally, Clare**
**13/3 Benton Avenue**
**Artarmon, NSW 2064(AU)**
Inventor: **FUNG, Ming-Chiu/Burton & Garran Hall**
**Australian National University**
**Acton, ACT 2601(AU)**

(74) Representative: **JENSEN & SON**
**70 Paul Street**
**London EC2A 4NA(GB)**

**Description**

The present invention relates to the molecular cloning of cDNA for murine interleukin-3, the use thereof, inter alia, as a hybridisation probe for eukaryotic interleukin-3, and for expression of biologically active murine interleukin-3.

DISCUSSION OF PRIOR ART

Interleukin-3 (IL-3) is one of a number of colony stimulating factors which are known to regulate haematopoiesis. The colony stimulating factors are hormone-like glycoproteins which are biologically active at very low levels. As they are produced in very minute quantities in vivo, they have proven to be very difficult to characterize by conventional biochemical methods. Also, due to their high level of activity, purification procedures have been hindered and although murine IL-3 derived from myelomonocytic leukaemia cell line WEHI-3 has been recently purified to apparent homogeneity (Ihle, J.N. et al, J.Immun. 131,282-287 (1983)), it was not necessarily clear at the time that the major protein in this preparation, from which the N-terminal sequence was derived was, in fact, murine IL-3.

IL-3 is produced by mitogen or antigen-activated T lymphocytes and by a number of continuous cell lines. It may also be produced by bone marrow cells. IL-3 is involved in regulating the growth and differentiation of pluripotent stem cells leading to the production of all major blood cell types. It has a broad range of biological activity and appears to be identical with a number of other factors named on the basis of their biological activities. These include multi-colony stimulating factor (CSF), haematopoietic growth factor, burst promoting activity, P-cell stimulating factor, mast cell growth factor, histamine-producing cell stimulating factor and Thy l-inducing activity.

It would be desirable to characterise this protein, as well as the DNA coding for the protein, so that large quantities of the protein could be produced for further study, as it is considered that it could play an important role in bone marrow transplantation, inter alia for the treatment of leukaemia.

As indicated above, murine IL-3, has only recently allegedly been purified to apparent homogeneity. However, as indicated above, the very high activity of the protein could lead to possible uncertainty about the N-terminal sequence reported and also synthetic DNA probes based on this sequence would have a high level of degeneracy which would preclude or substantially inhibit the molecular cloning of the DNA for the protein via the N terminal sequence data.

DESCRIPTION OF THE INVENTION

The present invention relates to the molecular cloning of cDNA for murine IL-3 and its use, inter alia, as a hybridisation probe and for expression of biologically active interleukin-3. The cDNA for murine IL-3 was obtained by a methodology as will be hereinafter described.

According to the present invention there is provided a method for the molecular cloning of murine interleukin-3 cDNA, the method comprising:

(i) culturing a cell line capable of producing murine interleukin-3 in culture medium;
(ii) isolating mRNA produced by the cultured cells;
(iii) fractionating the mRNA from step (ii) on the sucrose density gradient and identifying the fraction having interleukin-3 translation activity using an interleukin-3 dependent cell line;
(iv) preparing a cDNA library from the mRNA of step (iii) by synthesizing double-stranded cDNA and inserting the resultant cDNA into a suitable vector to generate a bank of recombinant DNA molecules, and transforming the cDNA library into a suitable host cell;
(v) pooling clones from the transformed cDNA library, culturing the pools and isolating DNA from the pooled clones, converting isolated plasmid DNA to a linear form with a suitable endonuclease, and removing or destroying any contaminating RNA,
(vi) hybridizing the DNA with mRNA isolated from a cell line capable of producing murine interleukin-3;
(vii) eluting bound mRNA and identifying the bound mRNA which has interleukin-3 translation activity using an interleukin-3 dependent cell line thereby identifying the clone possessing cDNA for murine interleukin-3; and
(viii) isolating and purifying cDNA for murine interleukin-3 from the clone identified in step (vii).

In another aspect the present invention provides cDNA for murine interleukin-3 in substantially pure form, or a DNA sequence complementary thereto.

In another embodiment the present invention provides a polynucleotide having a sequence equivalent to cDNA for murine interleukin-3 or part thereof as depicted in Figure 4 in substantially pure form. The invention further provides a double stranded DNA sequency, one strand of which is a polynucleotide as described above.

In another embodiment of the present invention there is provided a hybridization probe useful for identifying interleukin-3 DNA or RNA in eukaryotic cells, the probe comprising a polynucleotide having at least about ten bases, the polynucleotide having a sequence corresponding to cDNA of murine interleukin-3, or the DNA complementary thereto, or the double stranded DNA thereof, or RNA having a sequency corresponding to the mRNA sequence of murine interleukin-3, and a label attached to the polynucleotide, having a sequence substnatially equivalent to cDNA for murine interleukin-3 or part thereof as depicted in Figure 4 in substantially pure form.

Preferably said probe comprises the polynucleotide having a sequence corresponding to a cDNA of murine interleukin-3 or part thereof. Preferably the label is selected from a radioactive label or biotin.

Preferably the polynucleotide comprises at least fifteen bases.

In a further aspect of the present invention there is provided a method for identifying interleukin-3 DNA or RNA in eukaryotic cells or tissue sections or extracts thereof characterised in that the probe of the present invention is used.

A further aspect of the present invention is that a method for producing biologically active murine interleukin-3 or part thereof having an amino acid sequence substantially as depicted in Figure 4, the method comprising:

(i) extracting high molecular weight DNA from murine tissue or cultured cells, digesting the DNA to generate DNA fragments, size fractioning the DNA fragments and cloning the fragments into a suitable phase vector, transfecting a suitable host with the recombinant phases, identifying clones containing murine interleukin-3 encoding DNA by hybrodization with a probe according to claim 10, and isolating and purifying the murine interleukin-3 encoding DNA identified with the probe;

(ii) inserting the isolated and purified DNA into an appropriate cloning vector such that either the coding region for murine interleukin-3 is in phase with a prokaryotic protein or that the coding region for murine interleukin-3 is under the control of a prokaryotic promoter within the vector, to result in a recombinant cloning vector; and

(iii) transforming prokaryotic cells with the recombinant cloning vector and culturing the cells to produce murine interleukin-3.

The present invention also provides murine IL-3 in substantially pure form as a fusion protein or as a non-fusion protein. These proteins have N-terminal amino acids as depicted in Figure 8 of the accompanying drawings. Although these proteins are not glycosylated they are still biologically active.

## DESCRIPTION OF PREFERRED EMBODIMENTS

The invention will be further described by reference to the following drawings wherein:

Figure 1 depicts the interleukin-3 translation activity of mRNA from WEH1-3 cells, fractionated on a sucrose density gradient;

Figure 2 shows the titration curves for IL-3 activity in oocyte translates;

Figure 3 depicts the cDNA insert for pILM3 showing relevant restriction sites as well as the sequencing strategy;

Figure 4 depicts the nucleotide sequence for cDNA insert of pILM-3 and amino acid sequence of IL-3;

Figure 5 is a restriction map of vector pBTA 611;

Figure 6 is a restriction map of vector pBTA 610;

Figure 7 is a restriction map of vector pBTA 640.

Figure 8 depicts the N-terminal sequence of the proteins expressed by using vectors of Figs 5-7;

Figure 9 depicts schematically the murine IL-3 gene and

Figure 10 depicts the nucleotide sequence of the murine IL-3 gene and flanking regions.

The invention will be further described also, with reference to the following methodology.

## Translation of IL-3 mRNA in oocytes

Two different murine cell lines were compared as sources of IL-3 mRNA. The myelomonocytic line WEHI-3 is known to produce IL-3 constitutively (Lee, J.C., Hapel, A.J. & Ihle, J.N., J. Immun. 128, 2393-2398 (1982)) whereas the lymphoma line EL-4 produces IL-3 after stimulation with phorbol myristate acetate (PMA) (Ihle, J.N., Rebar, L., Keller, J., Lee, J.C. & Hapel, A.J.; Immun. Rev. 63, 5-32 (1982)). Messenger RNA was prepared from both cell lines using the guanidine thiocyanate method (McCandliss, R., Sloma, A & Pestka, S.; Meth. Enzym. 79, 51-59 (1981); Chirgwin, J.M., Przybyla, A.E., MacDonald, R.J. & Rutter, W.J., Biochemistry 18, 5294-5299 (1979)) and attempts were made to translate the mRNAs by microinjection into X.laevis oocytes (Sloma, A., McCandliss, R. & Pestka, S. Meth. Enzym. 79, 68-71 (1981)). The translates were assayed for biologically active IL-3 using the IL-3 dependent cell line 32D c1-23 (Ihle, J.N. et al. J. Immun. 129, 1377-1383 (1982)). It was found that mRNA from EL-4 could be translated in the oocytes to give readily detectable levels of IL-3 after 48 h incubation as shown in Table 1. Most of the detectable activity was present in the incubation medium indicating that the oocytes were secreting mature IL-3. Assays of oocyte extracts showed that they were inhibitory. mRNA from WEHI-3 cells regularly gave higher levels of IL-3 in the translation assays than preparations from EL-4 (Table 1), in keeping with the higher levels of IL-3 found in culture supernatants of the WEHI-3 cell line (data not shown).

TABLE 1

| Translation of IL-3 mRNA in Xenopus laevis oocytes | | | | |
|---|---|---|---|---|
| mRNA | Incubation time | IL-3 activity (c.p.m.) | | |
| | | Oocyte | Medium | Total extract |
| EL-4 | 24h | 0 | 3,100 | 3,700 |
| | 48h | - | 20,000 | 5,600 |
| WEHI-3 | 48h | 6,000 | 47,000 | 28,700 |

Assay for translation in oocytes

EL-4 cells were grown to a cell density of $10^6$ per ml in spinner cultures in DMEM medium supplemented with 10% fetal calf serum. The cells were collected by centrifugation, resuspended in serum-free medium containing 100 ng $ml^{-1}$ PMA and subcultured into $150cm^2$ culture flasks at 5 x $10^8$ cells per flask. The cells were incubated for 10 h before collection. WEHI-3 cells were grown to a density of $10^6$ per ml in RPMI-1640 medium supplemented with 10% fetal calf serum and collected by centrifugation. Afterwards, both cell pellets were resuspended in 0.1 volume of serum-free medium and mRNA was prepared by the guanidine thiocyanate-CsCl method (McCandliss, R., Sloma, A. & Pestka, S. Meth. Enzym. 79, 51-59 (1981); Chirgwin, J.M., Przybyla, A.E., MacDonald, R.J. & Rutter, W.J., Biochemistry 18, 5294-5299 (1979)). The mRNAs were further purified by chromatography on oligo(dT)-cellulose (Gray, P.W. et al, Nature 295, 503-508 (1982)) and after ethanol precipitation, were resuspended in buffer (10mM Tris-HCl, 0.1mM EDTA, pH 7.5) at a concentration of $lugul^{-1}$. The mRNAs were injected into 15 X. laevis oocytes ( 50nl per oocyte) according to standard methods (Sloma, A., McCandliss, R. & Pestka, S. Meth. Enzym. 79, 68-71 (1981)). After incubation, the medium was withdrawn and an extract of the oocytes prepared (Sloma, A., McCandliss, R. & Pestka, S. Meth. Enzym. 79, 68-71 (1981)). The incubation medium, the oocyte extract and a mixture of the two were assayed for IL-3 activity using the IL-3 dependent cell line 32D c1-23 (Ihle, J.N. et al, J. Immun. 129, 1377-1383 (1982)) according to the methods described previously, measuring incorporation of $^3$H-thymidine as an index of cell growth.

Hybrid plasmids containing IL-3 cDNA

IL-3 mRNA from WEHI-3 was fractionated on a sucrose density gradient and the fractions assayed by oocyte translation (Fig. 1). The major peak of activity sedimented at 12S. This procedure resulted in 10-20-fold purification of the IL-3 mRNA. RNA from the peak fractions was used as a template to synthesize double-stranded cDNA using the method of Land et al (Land, H., Grez, M., Hauser, H., Lindenmaier, W. & Schutz, G., Nucleic Acids Res. 9, 2251-2266 (1981)). The resultant cDNA was inserted into pAT153 by G-C tailing (Michelson, A.M. & Orkin, S.H., J. Biol. Chem. 257, 14773-14782 (1982)) and the cDNA library transformed into strain HB101. Approximately 5,000 individual clones were isolated. Of a small sample tested, 92% were ampicillin sensitive indicating the presence of cDNA inserts in the majority of the clones. Since the cDNA was not size fractionated prior to cloning, the sizes of inserts in the cDNA library varied from about 200 to 1,000 base pairs (bp). It was expected that even the short inserts would give satisfactory results in the mRNA hybridization-translation assay (see below).

Clones were grown in pools of 10 and DNA prepared by the method of Holmes and Quigley (Holmes, D.E. & Quigley, M., Anal. Biochem. 114, 193-197 (1981)). The plasmids were converted to a linear form with BamHI restriction endonuclease, contaminating RNA was destroyed by alkaline hydrolysis and the DNA was bound to nitrocellulose filters. The filter-bound DNA was hybridized with WEHI-3 mRNA and bound mRNA eluted and translated in oocytes. One positive pool of 10 clones was detected. The activity observed in translates derived from the positive pool was quite low but was consistently above the background and the values of the other clone pools. DNA was therefore prepared from the individual clones of the pool and the mRNA hybridization translation assay repeated. One of the clones (pILM1) was clearly positive (Table 2). The IL-3 activity detected showed the same titration curve as oocyte translates of WEHI-3 mRNA (Fig. 2).

Nucleotide sequence analysis

The cDNA insert in pILM1 was quite short (139 bp excluding the G-C tails). It was subcloned into the SmaI site of M13mp8 after shortening the G-C tails with Bal 31 exonuclease. The DNA sequence was determined on both strands using the chain termination method (Sanger, F., Nicklen, S. & Coulson, A.R. Proc. Natl. Acad Sci. U.S.A. 74, 5463-5467 (1977); Sanger, F., Coulson, A.R., Hong, G.F., Hill, D.F. & Petersen, G.B., J. Molec. Biol. 162, 729-773 (1982)). An oligonucleotide primer corresponding to nucleotides 92-112 of this cDNA insert was synthesized (Tanaka, T. & Letsinger, R.L., Nucleic Acids Res. 10, 3249-3260 (1982); McBride, L.J. & Caruthers, M.H., Tetrahedron Lett. 24, 245-248 (1983)) and labelled using [ -$^{32}$P]-dATP and polynucleotide kinase (Goodman, H.M. & MacDonald, R.J., Meth. Enzym. 68, 75-90 (1979)). The labelled probe was used to examine the remaining clones in the cDNA library (Grunstein, M. & Hogness, D.S. Proc. Natl. Acad. Sci. U.S.A. 72, 3961-3965 (1975); Wallace, R.B. et al, Nucleic Acids Res. 6, 3543-3557 (1979)). No other clones containing this sequence were detected.

To isolate a long clone encoding IL-3, a further cDNA library was prepared, but in this case the double-stranded cDNA was size fractionated using Sepharose CL-4B to enrich for cDNA fragments larger than 500bp. The resulting cDNA was inserted into pAT153 by G-C tailing and transformed into HB101. The labelled oligonucleotide probe described above was used to identify a longer cDNA clone (pILM3) which was shown by DNA sequencing to carry an insert of 629bp excluding the G-C tails. The DNA sequence of clone pILM3 was determined on both strands using the chain termination method (Sanger, F., Nicklen, S. & Coulson, A.R., Proc. Natl. Acad. Sci. U.S.A. 74, 5463-5467 (1977); Sanger, F., Coulson, A.R., Hong, G.F., Hill, D.F. & Petersen, G.B., J. Molec. Biol. 162, 729-773 (1982)) after subcloning into M13mp8 and 9. The sequencing strategy is in Fig. 3. The subcloning was complicated by the fact that one of the PstI sites flanking the cDNA insert had been lost due to exonucleolytic removal of one nucleotide during the G tailing of the vector. Clone pILM3 was found to extend beyond pILM1 at the 3' end only, both clones possessing the same 5' end. The DNA sequence is shown in Figure 4 and shows a single large open reading frame. Assuming that the first ATG from the 5' end serves as the initiation codon, the reading frame extends from nucleotides 28-525 inclusive and specifies a polypeptide of 166 amino acids. The 5' untranslated region is therefore quite short (27bp). The method of Land et al (Land, H., Grez, M., Hauser, H., Lindenmaier, W. & Schutz, G., Nucleic Acids Res. 9, 2251-2266 (1981)), which was used to generate the cDNA clones, is designed to produce a high proportion of cDNA clones with complete 5' termini. Since both cDNA clones isolated in the course of this work had the same 5' end, it is possible that pILM3 carries a close to full length copy of the 5' untranslated region of IL-3 mRNA. Clone pILM3, however, does not extend to the 3' end of the IL-3 mRNA and is probably missing a significant portion of the 3' untranslated region. The presence of the complete coding sequence for IL-3 is the important feature of the present invention.

TABLE 2

| mRNA hybridization translation assay for detection of IL-3 cDNA clones | |
|---|---|
| DNA sample | IL-3 activity (units per ml) |
| 1 | 1 |
| 2 | 1 |
| 3 | 1 |
| 4 | 1 |
| 5 | 1 |
| 6(pILM1) | 121,106,139 |
| 7 | 1 |
| 8 | 1 |
| 9 | 1 |
| 10 | 1 |

The reading frame specifies a protein of MW 18,540 as depicted by the single letter amino acid notation in Figure 4. The strongly hydrophobic stretch of 9 amino acids (residues 13-21) is indicative of the presence of a signal peptide. In the cases studied to date, the last residue of the signal sequence is invariably an amino acid with a small uncharged side chain. Analysis of the signal peptide region suggests a number of potential sites for cleavage by the signal peptidase. Application of the empirical rules proposed by Von Heijne (Von Heijne, G,Eur. J. Biochem. 133, 17-21 (1983)) predicts the end of the signal peptide at residue 27 (Ala). This potential cleavage site is shown with an arrow in Fig. 4.

The published N-terminal amino acid sequence of murine IL-3 (Ihle, J.N., Contemp. Topics Molec. Immun. (in the press)) is in complete agreement with the reading frame and extends from residues 33 (Asp) to 47 (Val). It seems most unlikely that the signal peptide would end at residue 32 (Arg) and this suggests that the murine IL-3 isolated from the WEHI-3 cell line has been further processed.

The protein sequence of mature processed IL-3 indicates a MW of 15,102 with four potential N-glycosylation sites (Asn-X-Ser or Asn-X-Thr). The four sites are underlined in Fig. 4. One of these is in the region of the published N-terminal sequence and glycosylation at this site would explain the inability to identify the Asn residue during automated Edman degradation. When the amino acid composition figures for murine IL-3 are normalized to the size predicted by the DNA sequence they give good agreement with the predicted composition (See Table 3 below). These methods enable production of large quantities of IL-3 by prokaryotic cells. The IL-3 produced is not glycosylated but still demonstrates high biological activity.

TABLE 3

| Amino acid composition of murine IL-3 | | |
|---|---|---|
| | Predicted | Determined |
| Phe | 5 | 4.2 |
| Leu | 15 | 16.7 |
| Ile | 5 | 4.8 |
| Met | 1 | 1.2 |
| Val | 10 | 8.3 |
| Ser | 13 | 11.3 |
| Pro | 10 | 7.1 |
| Thr | 8 | 7.1 |
| Ala | 3 | 4.2 |
| Tyr | 2 | 1.8 |
| His | 2 | 2.4 |
| Glu, Gln | 12 | 13.7 |
| Asp, Asn | 18 | 16.1 |
| Lys | 9 | 9.5 |
| Cys | 4 | ND |
| Trp | 0 | ND |
| Arg | 11 | 9.5 |
| Gly | 6 | 8.3 |

The amino acid sequence of murine IL-3 was compared with the sequences of human interleukin-2 and human immune interferon (Gray, P.W. et al., Nature 295, 503-508 (1982)) both of which are also produced by antigen-activated T lymphocytes. No significant sequence homology was detected. Although human IL-2 is of very similar size to IL-3 it carries no potential sites for N-glycosylation and does not appear closely related.

Use of murine IL-3 cDNA as hybridization probe General Methods

High mol. wt. DNA was prepared from mouse liver or cultured cells by gentle homogenization and treatment with Sarkosyl and proteinase K followed by equilibrium centrifugation in CsCl-ethidium bromide gradients.

DNA fragments were isolated by using electrophoresis on low-melting-point agarose gels (Wieslander, L. (1979) Anal. Biochem, 98, 305-309) or by using DEAE-membrane (Schleicher & Schull) in conjunction with normal agarose gels. Bacteriophage lambda DNA minipreparations from plate lysates were carried out as described (Maniatis et al., 1982 Molecular Cloning: A Laboratory Manual, Cold Spring Harbour Laboratory, NY, USA) except that the plates used were LM (Hanahan, D. (1983) J. Mol. Biol. 166, 557-580) containing agarose instead of agar. The phage DNA pellet was redissolved in 100 ul TE containing 50 ug/ml RNase, incubated at 37° for 20 min, extracted with phenol phenol/$CHCl_3$(1:1), then $CHCl_3$, and recovered by ethanol precipitation. The DNA was then redissolved in 50 ul of 10mm Tris/HCl, 0.1mM EDTA, pH 8.0. Plasmid DNA minipreparations were made by the rapid boiling method (Maniatis et al., 1982 supra) except that after RNase treatment, the DNA was further purified as described above for phage DNA minipreparations. Large scale plasmid DNA preparations were made by the method of Clewell & Helinski (1969, Proc. Natl. Acad. Sci. USA, 62. 1159-1166) following amplification with chloramphenicol. Isolated EcoRI fragments from recombinant bacteriophage were ligated to pAT153 which had been cleaved with EcoRI and dephosphorylated with calf intestinal alkaline phosphatase (Maniatis, 1982 supra) Transformation of *E. coli* strain <600 ($r_k$-, $m_k^+$ ) with ligation mixes was accomplished by the method of Hanahan (1983). The synthetic random 10-mer used as a primer for probe synthesis was prepared by the phosphoramidite method (Tanaka & Letsinger (1982) Nucleic Acids Res. 10, 3249-3260) using an equimolar mixture of all four bases at each position.

Nucleic acid hybridization

Agarose gel electrophoresis and transfer of DNA to nitrocellulose were performed by standard methods (Maniatis et al., 1982 supra). Nitrocellulose replicas of plates containing bacteriophage lambda plaques were prepared for hybridization as described by Benton & Davis (1977) (Science (Wash.), 196, 180-182). Binding of DNA to nitrocellulose for filter hybridization assay was accomplished essentially as described by Kafatos et al. (1979) Nucleic Acids Res. 1, 1541-1552). Conditions for the various hybridizations were as follows.

Southern hybridization: prehybridizaton (4h) and hybridization (18h) at 65° in 6xSSc, 5x Denhardt's solution (Denhardt. 1966). 100 ug/ml sonicated, denatured salmon sperm DNA. 0.5% (w/v) SDS and 10mM EDTA; washing. 10 min at room temperature and 60 min at 65° in 2xSSC.

Filter hybridization assays of DNA samples: prehybridization (4-6h) and hybridization (18h) as for Southern hybridization except that 5xSSC and 50mM sodium phosphate buffer, pH 6.8 replaced the 6xSSC, and the 1mM ATP and 1mM sodium pyrophosphate were included; washing. 20 min at room temperature in 2 changes of 2xSSC, 0.5% SDS and then 120 min at 65° in 0.1xSSC, 0.5% SDS followed by a further 30 min. under the same conditions in fresh buffer.

Plaque hybridization: prehybridization (5h) and hybridization (16h) at 42° in 50% (v/v) formamide, 5xSSPE. 5x Denhardt's solution. 100 ug/ml sonicated, denatured salmon sperm DNA, 0.1% (w/v) SDS, 1mMATP and 1mM sodium pyrophosphate; washing, 20-30 min at room temperature in 4 changes of 2xSSC, 0.1% SDS then 3h at 67° in 2 changes of 1xSSC, 0.1% SDS.

Hybridization in situ in dried agarose gels was performed by a modification of published methods. Briefly, agarose gels following electrophoresis of DNA fragments were soaked in 1mM $MgSO_4$ 0.5 ug/ml ethidium bromide and then dried between sheets of cellophane on a commercial gel dryer at 60° (2h). The gel/cellophane sandwich was wetted with water, and the separated gel was incubated for 20 min in 0.5N NaOH, 1 M NaCl then neutralized for 20 min in 0.5 M Tris/HCl pH 7.4, 1 M NaCl. The gel was then added directly to reserved hybridization liquid from the previous plaque screening and hybridization was allowed to proceed at 42° for 16h. Washing was for 10 min at room temperature in 2 changes of 2xSSC, 0.5% SDS, for 60 min at 60° in 4 changes of 2xSSV, 0.1% SDS, and then for 60 min at 60° in 4 changes of 0.1xSSC, 0.1% SDS.

All hybridization and washing of filters or dried gels at elevated temperatures was performed with continual agitation. Autoradiography was performed using Kodak XAR-5 or Fuji RX film at -70° with intensifying screens.

## Probe Synthesis

Probes were prepared by primed synthesis using the 467 bp HindIII-NcoI fragment of the IL-3 cDNA-containing plasmid pILM-3 as template and a synthetic random 10-mer as primer. A typical small scale reaction contained, in 20 ul, 100ng of template DNA and 1 ug of primer which had been heated together at 100° for 3 min and snap chilled to 0°, 50mM sodium phosphate buffer pH 6.8, 10mm $MgCl_2$ 10mM DTT, 500 uM DGTP, 500 uM DCTP, 500 uM TTP, 3 uM (variable) [$^{32}$P]-dATP (50 uCi), and 2.5 units of E. coli DNA polymerase I large fragment. The reaction was allowed to proceed for 2h at 47°. Incorporation into acid precipitable material (Maniatis et al, Molecular cloning: A Laboratory Manual: Cold Spring Harbour Laboratory, NT, USA, 1982) was 70%. For some Southern blots, the probe DNA was separated from low-molecular-weight material by chromatography on Sephadex G-50 fine (Comb et al. (1983) DNA, 2, 213-229). Generally, the probe was used without separation by including ATP (1mM) and sodium pyrophosphate (1 mM) in the prehybridization and hybridization fluid (Ullrich et al. (1984) EMBO J., 3, 361-364). Probe DNA was denatured before use by heating for 5 min at 100° and snap chilling. For library screening, the probe synthesis was scaled up and the probe was used at a concentration of $1x10^6$ cpm/ml (specific activity: $4x10^8$ cpm/ug cDNA). For Southern hybridization and filter hybridization of agarose gel fractions, probes were used at a concentration of $4x10^6$ cpm/ml (specific activity $8x10^8$ cpm/ug cDNA).

## Southern hybridization of murine genomic DNA

To assess the number of copies of the IL-3 gene present in the murine genome, DNA from different mouse strains was digested with EcoRI and subjected to agarose gel electrophoresis, transfer to nitrocellulose membrane and hybridization to a [$^{32}$P]-labelled probe derived from the 467 bp HindIII-NcoI fragment covering most of the coding portion of the murine IL-3 cDNA as described above. A single hybridizing fragment of appropriate intensity for a single copy gene was observed in all strains tested. The size of the genomic EcoRI fragment varied from 9kb for Balb/c and some other strains to 16kb for New Zealand Black. DNA from Balb/c and New Zealand Black gave identical results with BamHI, bands of 2.7kb and 10kb being observed. With HindIII, a single band of 6.4kb was observed in both cases. These results are consistent with the presence of a single copy of the IL-3 gene in the haploid mouse genome. The presence of two bands in the case of BamHI is due to the presence of a BamHI site known to lie between HindIII and NcoI sites in the cDNA which delimit the probe fragment used. A single band is obtained with HindIII, in spite of the presence of a HindIII site in the cDNA since this HindIII site was used to excise the HindIII-NcoI fragment used to synthesize the probe. Thus, only DNA to one side of this HindIII site will hybridize with the probe.

Genomic DNA from several cultured murine cell lines was examined by Southern hybridization. WEHI-3 ($D^-$), WEHI-3 ($D^+$) and A4A showed a band at 9kb as for Balb/c and an additional band, of similar intensity, at 4kb. B6, the original cell line from which the WEHI lines were derived, had a single band 9kb.

Cloning of the IL-3 gene fron Balb/c mice

Genomic DNA from Balb/c liver was digested to completion with EcoRI, size fractionated on gels and a fraction enriched for the IL-3 gene cloned into λgtWES. B. The lambda library was screened by plaque hybridization (Benton W.P. & Davis R.W. (1977) Science (Wash) 196, 180-182) using the IL-3 cDNA-derived probe.

Eight positive plaques were obtained from a total of 70,00 plaques. Six of these were plaque purified and phage DNA minipreparations were prepared and subjected to restriction endonuclease analysis. All six lambda clones contained an 8.6 kb EcoRI insert and gave identical patterns after double digestion with EcoRI/BamHI and EcoRI/HindIII. EcoRI excises the inserts from the phage arms, so that the orientation of the inserts would not affect the pattern in these double digests.

To facilitate further work, the 8.6 kb insert from one of the recombinant phages was recloned into the EcoRI site of pAT153 (Twigg & Sherratt, (1980) Nature 283, 216-218). This plasmid was designated pILM11 and was further analysed by restriction endonuclease digestion and agarose gel electrophoresis of plasmid DNA minipreparations. In situ hybridization of restriction fragments to the CDNA probe was used to localize the IL-3 gene to a 3.7 kb HincII-EcoRI fragment (Figure 9). This fragment was isolated by low-melting point agarose gel electrophoresis of a HincII-EcoRI digest of pILM11 plasmid DNA.

Nucleotide sequence of the murine IL-3 gene

The nucleotide sequence of a 3490 bp region of the 3.7 kb HincII-EcoRI fragment containing the gene was determined on both strands by the chain-termination method (Sanger et al., Proc. Natl. Acad. Sci. U.S.A., 74, 5463-5467 (1977)). Figure 9 shows a summary of some of the gel readings used to assemble the sequence. The sequence of this region is presented in Figure 10.

Organization of the IL-3 gene

The IL-3 gene is made up of five exons interrupted by four introns. This arrangement is shown schematically in Figure 9 and is indicated in Figure 10.

The sequence of the five exons is in complete agreement with our previously determined cDNA sequence. In particular, nucleotide 2302 is A (Figure 10) as is the corresponding nucleotide 463 in the cDNA. Yokota et al. (1984 Proc. Natl. Acad. Sci. U.S.A., 81, 1070-1074) have reported that this nucleotide is G in their cDNA clone which would change the encoded amino acid from Thr to Ala. Whether this change represents allelic variation or some artefact arising during cDNA synthesis is not known. The predicted amino acid sequence of IL-3 from the WEHI-3 cell line, the source of mRNA for our cDNA and from normal Balb/c mice, the source of genomic DNA in the present work is identical.

Transcriptional control signals

Transcription of the IL-3 gene almost certainly begins at nucleotide 494 indicated in Figure 10. Several cDNA clones obtained by two different groups by methods likely to preserve the 5' terminus of the mRNA intact terminate at this position. A potential TATA box, is located upstream from the start of transcription (Figure 10). The distance between the TATA sequence and the start of transcription is 27 nucleotides, similar to the mean distance observed for eukaryotic promoters.

Expression of biologically active murine interleukin-3

Interleukin-3 was expressed in E. coli as a functionally active protein by two approaches. Firstly as fusion proteins with the first few amino acids of B-galactosidase and secondly as a non fusion protein corresponding to one of the putative mature protein sequences. The first amino acid of the mature protein is still unclear since aspartate, amino acid 33 (Ihle, J.N. et al., J. Immun. 131, 282-287, 1983) as well as Alanine, 9.9.27) have been reported to be the N-terminal amino acid. Therefore in the construction of IL-3 a number of start points were chosen.

Construction of IL-3 Fusion Expression Clones

(i) pBTA611

This expression clone was designed to express a protein consisting of 10 amino acids of B-galactosidase fused to the IL-3 gene coding from Arginine, amino acid 32, to the end of the gene (see Figure 8).

Approximately 5 ug of pILM3 DNA was digested with HaeIII and the DNA fragments separated on a 6% polyacrylamide gel. pBR322 DNA digested with HaeIII was run as molecular weight markers. The 450bp HaeIII fragment from pILM3 was cut out of the gel and eluted in 300 ul of 500 mM Ammonium Acetate, 1 mM EDTA pH 8.0, 0.1% SDS at 37°C for 16 hours. The supernatant was ethanol precipitated, redissolved in 300 ul of 300 mM sodium acetate and ethanol precipitated again. The 450bp HaeIII fragment was ligated to Pst I linkers d(pGCTGCAGC), then digested with Pst I and electrophoresed on a 6% polyacrylamide gel. The HaeIII fragment with Pst I cohesive ends was eluted as described above, and then ligated with Pst I, digested and phosphatased pUC9 DNA (Vierira and Messing, Gene 19, 259, 1983). The ligation mix was transformed into the $lon^-$ strain NB42 and plated on L + Ampicillin plates. DNA was prepared from a number of colonies, digested with Pst I and electrophoresed on a 6% polyacrylamide gel.

The majority of clones had 450bp Pst I inserts. The orientation of the inserts was determined by BamHI digestion. In the correct orientation, where the IL-3 gene is in phase with the B-galactosidase coding region, a 290bp BamHI fragment is expected. Clones with this restriction profile, called BTA611 (Figure 5), were picked and assayed for expression of an IL-3 protein. BTA611 colonies were inoculated into 3 mls of L broth and incubated overnight with shaking at 37°C. Approximately 300 ul of culture was TCA precipitated and the proteins separated on a 15% polyacrylamide SDS gel. An extra 17 K dalton protein band was observed in the BTA611 track which did not appear in the control track of pUC9 in NB42.

(ii) BTA610

This expression clone was designed to express a protein consisting of 9 amino acids of B-galactosidase fused to IL-3 gene coding from Alanine, amino acid 27, to the end of the gene (see Figure 8).

Approximately 5 ug of pILM3 DNA was digested with HindIII and NcoI and end-filled with Klenow polymerase and the DNA fragments separated on a 6% polyacrylamide gel. The 457bp HindIII/NcoI fragment was eluted from the gel and ligated into the end-filled BamHI site of pUC8 (Vieira and Messing, Gene 19, 259, 1983). The ligation mix was transformed into a $lon^-$ strain NB42 and plated onto L + Ampicillin plates. DNA was prepared from a number of colonies and digested with HaeIII to observe the insert and EcoRI/BglII to determine orientation. Clones with the insert in the correct orientation, where the IL-3 gene is in phase with the B-galactosidase gene were called BTA610 (Figure 6). Cultures of BTA610 were assayed for the presence of a 17 K dalton protein by polyacrylamide SDS gel electrophoresis as described previously. A predominant protein band was observed in the BTA610 track at 17.7 K daltons and was not present in the control track of pUC8 in NB42.

Biological Activity of the IL-3 Expression Constructs

Cultures of BTA611, BTA610, pUC8 and pUC9 in NB42 were incubated at 37°C for 16 hours in 50 mls L broth and 50 ug/ml Ampicillin. The cell pellets were lysed by sonication and the supernatant from each culture assayed for biologically active IL-3 using the IL-3 dependent cell line 32D c1 = 23, (Ihle. J.N. et al., J. Immun. 129, 1377-1383, 1982). Both BTA610 and BTA611 culture sonicates demonstrated high levels of biologically active IL-3, (2 x $10^4$ units/ml) and both pUC8 and pUC9 were negative for IL-3 activity.

Construction of IL-3 Non Fusion Expression Clones

(i) pBTA640

This expression clone was designed to express mature IL-3 from Alanine at amino acid 27 to the end of the gene, under the control of the tac promoter (Amann et al., Gene 25, 167-178. 1983).

ptac12 was digested with PvuII and ligated with a 22mer linker d(pAAATGGCTTCAATCAGTGGCCA). This linker includes the ATG start codon and amino acids 27-31 of IL-3. The last 6bp of the linker is a BalI site. The ptac12 linker ligation mix was transformed into MC1061 and plated onto L+Ampicillin plates. DNA from a number of colonies were digested with BalI and EcoRI to verify the cloning of the linker molecules and linker orientation. DNA from the correct construction with the ATG 5' to the IL-3 codons, was then digested with BalI and phosphatased and ligated with the 450bp HaeIII fragment from pILM-3. The ligation mix was digested with BalI. since a BalI site is only created when the HaeIII fragment is in the non-coding orientation. The ligation mix was then transformed into lon$^-$ NB42 F$^1$ (lacIq) and plated onto L+Ampicillin plates. DNA from a number of colonies was prepared and digested with EcoRI/BamHI to verify cloning of the HaeIII fragment and its orientation. The majority of clones had the HaeIII fragment in the correct orientation where the IL-3 coding region was in phase with the linker coding region. These clones were called BTA640 (Figure 7). BTA640 colonies were inoculated into 3 mls of M9 broth and induced at an $OD_{600}$ of 0.6 with 0.2% lactose for 3 hours. Approximately 300 ul of culture was TCA precipitated and the proteins separated on a 15% polyacrylamide gel. An extra protein band at 16.8 K daltons was observed in the BTA640 track which did not appear in the control track of ptac12 in NB42 F$^1$.

DNA transfer vectors and expression

The cDNA insert encoding IL-3 was excised from the plasmid pILM3, made blunt ended, and inserted into the SV40 shuttle vector pSV2neo[37] at the unique Hind III site using standard methods. Insertion of the coding sequence into pSV2neo places the expression of IL-3 under the control of the SV40 early promoter. One of the resultant recombinant plasmids (designated pILM4) was shown to have the insert in the correct orientation by restriction enzyme digestion. The plasmid DNA was transformed into monkey COS cells. After 48 hrs growth it was shown that the culture medium contained good levels of IL-3, assayed using IL-3 dependent cell lines.

This method enables the production of IL-3 by mammalian cells.

**Claims**

**1.** A method for the molecular cloning of murine interleukin-3 cDNA, the method comprising:
(i) culturing a cell line capable of producing murine interleukin-3 in culture medium;
(ii) isolating mRNA produced by the cultured cells;
(iii) fractionating the mRNA from steP (ii) on a sucrose density gradient and identifying the fraction having interleukin-3 translation activity using an interleukin-3 dependent cell line;
(iv) preparing a cDNA library from the mRNA of step (iii) by synthesizing double-stranded cDNA and inserting the resultant cDNA into a suitable vector to generate a bank of recombinant DNA molecules, and transforming the cDNA library into a suitable host cell;
(v) pooling clones from the transformed cDNA library, culturing the pools and isolating DNA from the pooled clones, converting isolated plasmid DNA to a linear form with a suitable endonuclease, and removing or destroying any contaminating RNA,
(vi) hybridizing the DNA with mRNA isolated from a cell line capable of producing murine interleukin-3;
(vii) eluting bound mRNA and identifying the bound mRNA which has interleukin-3 translation activity using an interleukin-3 dependent cell line thereby identifying the clone possessing cDNA for murine interleukin-3; and
(viii) isolating and purifying cDNA for murine interleukin-3 from the clone identified in step (vii).

**2.** cDNA prepared from the method of claim 1.

**3.** A polynucleotide having a sequence equivalent to cDNA for murine interleukin-3 or part thereof as depicted in Figure 4 in substantially pure form.

**4.** A polynucleotide complementary to the polynucleotide of claim 3.

**5.** A polynucleotide comprising RNA corresponding to the polynucleotide of claim 3 or claim 4.

**6.** Murine interleukin-3 in substantially pure form and having an amino acid sequence as depicted in Figure 4 of the accompanying drawings.

**7.** A hybridization probe useful for identifying interleukin-3 DNA or RNA in eukaryotic cells, the probe comprising a polynucleotide having at least about ten bases, the polynucleotide having a sequence corresponding to cDNA of murine interleukin-3, or the DNA complementary thereto, or the double stranded DNA thereof, or RNA having a sequence corresponding to the mRNA sequence of murine interleukin-3, and a label attached to the polynucleotide, having a sequence substantially equivalent to cDNA for murine interleukin-3 or part thereof as depicted in Figure 4 in substantially pure form.

**8.** The probe of claim 7 wherein the label is selected from a radioactive label or biotin.

**9.** The probe of claim 7 or 8 wherein the polynucleotide comprises at least fifteen bases.

**10.** The probe of any one of claims 7 to 9 wherein the polynucleotide has a sequence corresponding to a cDNA of murine interleukin-3 or part thereof.

**11.** A method for identifying interleukin-3 DNA or RNA in eukaryotic cells or tissue sections or extracts thereof characterized in that the probe of any one of claims 7 to 10 is used.

**12.** cDNA for murine interleukin-3 substantially as hereinbefore described and as depicted in Figure 4.

**13.** A method for producing biologically active murine interleukin-3 or part thereof having an amino acid sequence substantially as depicted in Figure 4, the method comprising:

(i) extracting high molecular weight DNA from murine tissue or cultured cells, digesting the DNA to generate DNA fragments, size fractionating the DNA fragments and cloning the fragments into a suitable phage vector, transfecting a suitable host with the recombinant phages, identifying clones containing murine interleukin-3 encoding DNA by hybridization with a probe according to claim 10, and isolating and purifying the murine interleukin-3 encoding DNA identified with the probe;

ii) inserting the isolated and purified DNA into an appropriate cloning vector such that either the coding region for murine interleukin-3 is in phase with a prokaryotic protein or that the coding region for murine interleukin-3 is under the control of a prokaryotic promoter within the vector, to result in a recombinant cloning vector; and

(iii) transforming prokaryotic cells with the recombinant cloning vector and culturing the cells to produce murine interleukin-3.

**14.** The method of claim 13 wherein the murine interleukin-3 is expressed as a fusion protein.

**15.** The method of claim 14 wherein the fusion protein comprises mature murine interleukin-3 and an oligopeptide comprising at least the first 9 amino acids of b-galactosidase.

**16.** The method of claim 13 wherein the murine interleukin-3 is expressed as a non-fusion protein.

**17.** The method of claim 16 wherein the the non-fusion protein begins at any of the first 32-40 amino acids of murine interleukin-3.

**18.** The recombinant clone pBTA610 which consists of: pUC8, digested with BamHI and end-filled with Klenow into which has been inserted the 4756p Hind III/Nco I fragment

```
                                                                                          A GCT TCA
                                                                                            Ala Ser

ATC AGT GGC CGG GAT ACC CAC CGT TTA ACC AGA ACG TTG AAT TGC AGC TCT ATT
Ile Ser Gly Arg Asp Thr His Arg Leu Thr Arg Thr Leu Asn Cys Ser Ser Ile

GTC AAG GAG ATT ATA GGG AAG CTC CCA GAA CCT GAA CTC AAA ACT GAT GAT GAA
Val Lys Glu Ile Ile Gly Lys Leu Pro Glu Pro Glu Leu Lys Thr Asp Asp Glu

GGA CCC TCT CTG AGG AAT AAG AGC TTT CGG AGA GTA AAC CTG TCC AAA TTC GTG
Gly Pro Ser Leu Arg Asn Lys Ser Phe Arg Arg Val Asn Leu Ser Lys Phe Val

GAA AGC CAA GGA GAA GTG GAT CCT GAG GAC AGA TAC GTT ATC AAG TCC AAT CTT
Glu Ser Gln Gly Glu Val Asp Pro Glu Asp Arg Tyr Val Ile Lys Ser Asn Leu

CAG AAA CTT AAC TGT TGC CTG CCT ACA TCT GCG AAT GAC TCT GCG CTG CCA GGG
Gln Lys Leu Asn Cys Cys Leu Pro Thr Ser Ala Asn Asp Ser Ala Leu Pro Gly

GTC TTC ATT CGA GAT CTG GAT GAC TTT CGG AAG AAA CTG AGA TTC TAC ATG GTC
Val Phe Ile Arg Asp Leu Asp Asp Phe Arg Lys Lys Leu Arg Phe Tyr MET Val

CAC CTT AAC GAT CTG GAG ACA GTG CTA ACC TCT AGA CCA CCT CAG CCC GCA TCT
His Leu Asn Asp Leu Glu Thr Val Leu Thr Ser Arg Pro Pro Gln Pro Ala Ser

GGC TCC GTC TCT CCT AAC CGT GGA ACC GTG GAA TGT TAA AACAGCAGGC AGAGCACCTA
Gly Ser Val Ser Pro Asn Arg Gly Thr Val Glu Cys

AAGTCTGAAT GTTCCTCATG GCCCATG
```

end-filled with Klenow.

**19.** The recombinant clone pBTA611 which consists of: pUC9, digested with Pst I, into which has been inserted the 450bp HaeIII fragment

```
        C CGG GAT ACC CAC CGT TTA ACC AGA ACG TTG AAT TGC AGC TCT ATT
          Arg Asp Thr His Arg Leu Thr Arg Thr Leu Asn Cys Ser Ser Ile

GTC AAG GAG ATT ATA GGG AAG CTC CCA GAA CCT GAA CTC AAA ACT GAT GAT GAA
Val Lys Glu Ile Ile Gly Lys Leu Pro Glu Pro Glu Leu Lys Thr Asp Asp Glu

GGA CCC TCT CTG AGG AAT AAG AGC TTT CGG AGA GTA AAC CTG TCC AAA TTC GTG
Gly Pro Ser Leu Arg Asn Lys Ser Phe Arg Arg Val Asn Leu Ser Lys Phe Val

GAA AGC CAA GGA GAA GTG GAT CCT GAG GAC AGA TAC GTT ATC AAG TCC AAT CTT
Glu Ser Gln Gly Glu Val Asp Pro Glu Asp Arg Tyr Val Ile Lys Ser Asn Leu

CAG AAA CTT AAC TGT TGC CTG CCT ACA TCT GCG AAT GAC TCT GCG CTG CCA GGG
Gln Lys Leu Asn Cys Cys Leu Pro Thr Ser Ala Asn Asp Ser Ala Leu Pro Gly

GTC TTC ATT CGA GAT CTG GAT GAC TTT CGG AAG AAA CTG AGA TTC TAC ATG GTC
Val Phe Ile Arg Asp Leu Asp Asp Phe Arg Lys Lys Leu Arg Phe Tyr MET Val

CAC CTT AAC GAT CTG GAG ACA GTG CTA ACC TCT AGA CCA CCT CAG CCC GCA TCT
His Leu Asn Asp Leu Glu Thr Val Leu Thr Ser Arg Pro Pro Gln Pro Ala Ser

GGC TCC GTC TCT CCT AAC CGT GGA ACC GTG GAA TGT TAA AACAGCAGGC AGAGCACCTA
Gly Ser Val Ser Pro Asn Arg Gly Thr Val Glu Cys

AAGTCTGAAT GTTCCTCATG G
```

ligated to Pst I linkers d(pGCTGCAGC) and digested with Pst I.

**20.** The recombinant clone pBTA640 consisting of: pta12, digested with PvuII and ligated with the 22mer linker d(pAAATGGCTTCAATCAGTGGCCA), digested with BalI and phosphatased and ligated with the 450bp HaeIII fragment

```
C CGG GAT ACC CAC CGT TTA ACC AGA ACG TTG
y Arg Asp Thr His Arg Leu Thr Arg Thr Leu

AAT TGC AGC TCT ATT
Asn Cys Ser Ser Ile

GTC AAG GAG ATT ATA GGG AAG CTC CCA GAA CCT GAA CTC AAA ACT GAT GAT GAA
Val Lys Glu Ile Ile Gly Lys Leu Pro Glu Pro Glu Leu Lys Thr Asp Asp Glu

GGA CCC TCT CTG AGG AAT AAG AGC TTT CGG AGA GTA AAC CTG TCC AAA TTC GTG
Gly Pro Ser Leu Arg Asn Lys Ser Phe Arg Arg Val Asn Leu Ser Lys Phe Val

GAA AGC CAA GGA GAA GTG GAT CCT GAG GAC AGA TAC GTT ATC AAG TCC AAT CTT
Glu Ser Gln Gly Glu Val Asp Pro Glu Asp Arg Tyr Val Ile Lys Ser Asn Leu

CAG AAA CTT AAC TGT TGC CTG CCT ACA TCT GCG AAT GAC TCT GCG CTG CCA GGG
Gln Lys Leu Asn Cys Cys Leu Pro Thr Ser Ala Asn Asp Ser Ala Leu Pro Gly

GTC TTC ATT CGA GAT CTG GAT GAC TTT CGG AAG AAA CTG AGA TTC TAC ATG GTC
Val Phe Ile Arg Asp Leu Asp Asp Phe Arg Lys Lys Leu Arg Phe Tyr MET Val

CAC CTT AAC GAT CTG GAG ACA GTG CTA ACC TCT AGA CCA CCT CAG CCC GCA TCT
His Leu Asn Asp Leu Glu Thr Val Leu Thr Ser Arg Pro Pro Gln Pro Ala Ser

GGC TCC GTC TCT CCT AAC CGT GGA ACC GTG GAA TGT TAA AACAGCAGGC AGAGCACCTA
Gly Ser Val Ser Pro Asn Arg Gly Thr Val Glu Cys

AAGTCTGAAT GTTCCTCATG G .
```

**21.** Murine interleukin-3 as depicted in Figure 4 in substantially pure form when produced by the method of any one of claims 13 to 17.

## Patentansprüche

**1.** Verfahren zum molekularen Klonieren von Mäuse-Interleukin-3-cDNA, mit den Schritten:

(i) Züchten einer zur Bildung von Mäuse-Interleukin-3 in einem Kulturmedium befähigten Zellinie;

(ii) Isolieren von von den gezüchteten Zellen gebildeter mRNA;

(iii) Fraktionieren der mRNA von Schritt (ii) auf einem Saccharose-Dichtegradienten und Identifizieren der Interleukin-3-Translations-Aktivität aufweisenden Fraktion unter Verwendung einer Interleukin-3-abhängigen Zellinie;

(iv) Herstellen einer cDNA-Bibliothek aus der mRNA von Schritt (iii) durch Synthese doppelsträngiger cDNA und Insertieren der resultierenden cDNA in einen geeigneten Vektor zur Erzeugung einer Bank von rekombinanten DNA-Molekülen sowie Transformieren der cDNA-Bibliothek in eine geeignete Wirtszelle;

(v) Poolen von Klonen aus der transformierten cDNA-Bibliothek, Züchten der Pools und Isolieren von DNA aus den gepoolten Klonen, Umwandlung von isolierter Plasmid-DNA in eine lineare Form mit einer geeigneten Endonuklease sowie Entfernen oder Zerstören von jeglicher kontaminierender RNA;

(vi) Hybridisieren der DNA mit mRNA, die aus einer zur Bildung von Mäuse-Interleukin-3 befähigten Zellinie isoliert worden ist;

(vii) Eluieren gebundener mRNA und Identifizieren der gebundenen mRNA, die Interleukin-3-Translations-Aktivität aufweist, unter Verwendung einer Interleukin-3-abhängigen Zellinie, wodurch der cDNA für Mäuse-Interleukin-3 aufweisende Klon identifiziert wird; und

(viii) Isolieren und Reinigen von cDNA für Mäuse-Interleukin-3 aus dem in Schritt (vii) identifizierten Klon.

**2.** cDNA, hergestellt nach dem Verfahren von Anspruch 1.

**3.** Polynukleotid, das eine mit cDNA für Mäuse-Interleukin-3 äquivalente Sequenz gemäß Fig. 4 oder einen Teil derselben aufweist, in im wesentlichen reiner Form.

**4.** Polynukleotid, das zu dem Polynukleotid von Anspruch 3 komplementär ist.

**5.** Polynukleotid, das dem Polynukleotid von Anspruch 3 oder 4 entsprechende RNA umfaßt.

**6.** Mäuse-Interleukin-3 in im wesentlichen reiner Form, die eine wie in Fig. 4 der beigefügten Zeichnungen gezeigte Aminosäuresequenz aufweist.

**7.** Hybridisierungs-Sonde, die zum Identifizieren von Interleukin-3-DNA oder -RNA in eukaryontischen Zellen verwendbar ist, wobei die Sonde ein Polynukleotid mit mindestens etwa zehn Basen und mit einer Sequenz, die cDNA von Mäuse-Interleukin-3, komplementärer DNA derselben, doppelsträngiger DNA derselben oder RNA mit einer der mRNA-Sequenz von Mäuse-Interleukin-3 analogen Sequenz entspricht, und einen mit dem Polynukleotid, das eine im wesentlichen zu cDNA für Mäuse-Interleukin-3 gemäß Fig. 4 oder einem Teil derselben äquivalente Sequenz aufweist, verknüpften Marker umfaßt, in im wesentlichen reiner Form.

**8.** Sonde nach Anspruch 7, bei der der Marker aus einem radioaktiven Marker oder Biotin ausgewählt ist.

**9.** Sonde nach Anspruch 7 oder 8, bei dem das Polynukleotid mindestens fünfzehn Basen umfaßt.

**10.** Sonde nach einem jeden der Ansprüche 7 bis 9, bei dem das Polynukleotid eine Sequenz aufweist, die einer cDNA von Mäuse-Interleukin-3 oder einem Teil derselben entspricht.

**11.** Verfahren zum Identifizieren von Interleukin-3-DNA oder -RNA in eukaryontischen Zellen oder Gewebeschnitten oder Extrakten derselben, dadurch gekennzeichnet, daß die Sonde nach einem jeden der Ansprüche 7 bis 10 verwendet wird.

**12.** cDNA für Mäuse-Interleukin-3, im wesentlichen wie hier beschrieben und in Fig. 4 gezeigt.

**13.** Verfahren zur Herstellung von biologisch aktivem Mäuse-Interleukin-3 oder einem Teil desselben, mit im wesentlichen einer Aminosäuresequenz wie in Fig. 4 gezeigt, mit den Schritten:
(i) Extrahieren von DNA mit hohem Molekulargewicht aus Mäusegewebe oder gezüchteten Zellen, Verdauen der DNA zur Erzeugung von DNA-Fragmenten, Fraktionieren der DNA-Fragmente nach der Größe und Klonieren der Fragmente in einen geeigneten Phagenvektor, Transfizieren eines geeigneten Wirts mit den rekombinanten Phagen, Identifizieren von Klonen, die Mäuse-Interleukin-3 kodierende DNA enthalten, durch Hybridisieren mit einer Sonde gemäß Anspruch 10 sowie Isolieren und Reinigen der Mäuse-Interleukin-3 kodierenden, mit der Sonde identifizierten DNA;
(ii) Insertieren der isolierten und gereinigten DNA in einen geeigneten Klonierungsvektor, so daß entweder die Kodierungsregion für Mäuse-Interleukin-3 sich in Phase mit einem prokaryontischen Protein befindet oder die Kodierungsregion für Mäuse-Interleukin-3 sich unter der Kontrolle eines prokaryontischen Promotors in dem Vektor befindet, um einen rekombinanten Klonierungsvektor zu ergeben; und
(iii) Transformieren prokaryontischer Zellen mit dem rekombinanten Klonierungsvektor und Züchten der Zellen zur Herstellung von Mäuse-Interleukin-3.

**14.** Verfahren gemäß Anspruch 13, bei dem das Mäuse-Interleukin-3 als Fusionsprotein exprimiert wird.

**15.** Verfahren gemäß Anspruch 14, bei dem das Fusionsprotein reifes Mäuse-Interleukin-3 und ein mindestens die ersten 9 Aminosäuren von b-Galactosidase umfassendes Oligopeptid umfaßt.

**16.** Verfahren gemäß Anspruch 13, bei dem das Mäuse-Interleukin-3 als ein Nicht-Fusions-Protein exprimiert wird.

**17.** Verfahren gemäß Anspruch 16, bei dem das Nicht-Fusions-Protein bei einer beliebigen der ersten 32 bis 40 Aminosäuren von Mäuse-Interleukin-3 beginnt.

**18.** Rekombinanter Klon pBTA610, der aus mit BamHI verdautem und am Ende mit Klenow aufgefülltem pUC8, in das das am Ende mit Klenow aufgefüllte 4756p-HindIII/NcoI-Fragment

```
                                                                      A GCT TCA
                                                                        Ala Ser

ATC AGT GGC CGG GAT ACC CAC CGT TTA ACC AGA ACG TTG AAT TGC AGC TCT ATT
Ile Ser Gly Arg Asp Thr His Arg Leu Thr Arg Thr Leu Asn Cys Ser Ser Ile

GTC AAG GAG ATT ATA GGG AAG CTC CCA GAA CCT GAA CTC AAA ACT GAT GAT GAA
Val Lys Glu Ile Ile Gly Lys Leu Pro Glu Pro Glu Leu Lys Thr Asp Asp Glu

GGA CCC TCT CTG AGG AAT AAG AGC TTT CGG AGA GTA AAC CTG TCC AAA TTC GTG
Gly Pro Ser Leu Arg Asn Lys Ser Phe Arg Arg Val Asn Leu Ser Lys Phe Val

GAA AGC CAA GGA GAA GTG GAT CCT GAG GAC AGA TAC GTT ATC AAG TCC AAT CTT
Glu Ser Gln Gly Glu Val Asp Pro Glu Asp Arg Tyr Val Ile Lys Ser Asn Leu

CAG AAA CTT AAC TGT TGC CTG CCT ACA TCT GCG AAT GAC TCT GCG CTG CCA GGG
Gln Lys Leu Asn Cys Cys Leu Pro Thr Ser Ala Asn Asp Ser Ala Leu Pro Gly

GTC TTC ATT CGA GAT CTG GAT GAC TTT CGG AAG AAA CTG AGA TTC TAC ATG GTC
Val Phe Ile Arg Asp Leu Asp Asp Phe Arg Lys Lys Leu Arg Phe Tyr MET Val

CAC CTT AAC GAT CTG GAG ACA GTG CTA ACC TCT AGA CCA CCT CAG CCC GCA TCT
His Leu Asn Asp Leu Glu Thr Val Leu Thr Ser Arg Pro Pro Gln Pro Ala Ser

GGC TCC GTC TCT CCT AAC CGT GGA ACC GTG GAA TGT TAA AACAGCAGGC AGAGCACCTA
Gly Ser Val Ser Pro Asn Arg Gly Thr Val Glu Cys

AAGTCTGAAT GTTCCTCATG GCCCATG
```

insertiert ist, besteht.

**19.** Rekombinanter Klon pBTA611, der aus mit PstI verdautem pUC9, in das das mit PstI-Linkern d-(pGCTGCAGC) ligasierte und mit PstI verdaute 450bp-HaeIII-Fragment

```
             C CGG GAT ACC CAC CGT TTA ACC AGA ACG TTG AAT TGC AGC TCT ATT
               Arg Asp Thr His Arg Leu Thr Arg Thr Leu Asn Cys Ser Ser Ile

GTC AAG GAG ATT ATA GGG AAG CTC CCA GAA CCT GAA CTC AAA ACT GAT GAT GAA
Val Lys Glu Ile Ile Gly Lys Leu Pro Glu Pro Glu Leu Lys Thr Asp Asp Glu

GGA CCC TCT CTG AGG AAT AAG AGC TTT CGG AGA GTA AAC CTG TCC AAA TTC GTG
Gly Pro Ser Leu Arg Asn Lys Ser Phe Arg Arg Val Asn Leu Ser Lys Phe Val

GAA AGC CAA GGA GAA GTG GAT CCT GAG GAC AGA TAC GTT ATC AAG TCC AAT CTT
Glu Ser Gln Gly Glu Val Asp Pro Glu Asp Arg Tyr Val Ile Lys Ser Asn Leu

CAG AAA CTT AAC TGT TGC CTG CCT ACA TCT GCG AAT GAC TCT GCG CTG CCA GGG
Gln Lys Leu Asn Cys Cys Leu Pro Thr Ser Ala Asn Asp Ser Ala Leu Pro Gly

GTC TTC ATT CGA GAT CTG GAT GAC TTT CGG AAG AAA CTG AGA TTC TAC ATG GTC
Val Phe Ile Arg Asp Leu Asp Asp Phe Arg Lys Lys Leu Arg Phe Tyr MET Val

CAC CTT AAC GAT CTG GAG ACA GTG CTA ACC TCT AGA CCA CCT CAG CCC GCA TCT
His Leu Asn Asp Leu Glu Thr Val Leu Thr Ser Arg Pro Pro Gln Pro Ala Ser

GGC TCC GTC TCT CCT AAC CGT GGA ACC GTG GAA TGT TAA AACAGCAGGC AGAGCACCTA
Gly Ser Val Ser Pro Asn Arg Gly Thr Val Glu Cys

AAGTCTGAAT GTTCCTCATG G
```

insertiert ist, besteht.

**20.** Rekombinanter Klon pBTA640, der aus ptac12, verdaut mit PvuII, ligasiert mit dem 22meren Linker d-(pAAATGGCTTCAATCAGTGGCCA), verdaut mit BalI, phosphatasiert und mit dem 450bp-HaeIII-Fragment

C CGG GAT ACC CAC CGT TTA ACC AGA ACG TTG
y Arg Asp Thr His Arg Leu Thr Arg Thr Leu

AAT TGC AGC TCT ATT
Asn Cys Ser Ser Ile

GTC AAG GAG ATT ATA GGG AAG CTC CCA GAA CCT GAA CTC AAA ACT GAT GAT GAA
Val Lys Glu Ile Ile Gly Lys Leu Pro Glu Pro Glu Leu Lys Thr Asp Asp Glu

GGA CCC TCT CTG AGG AAT AAG AGC TTT CGG AGA GTA AAC CTG TCC AAA TTC GTG
Gly Pro Ser Leu Arg Asn Lys Ser Phe Arg Arg Val Asn Leu Ser Lys Phe Val

GAA AGC CAA GGA GAA GTG GAT CCT GAG GAC AGA TAC GTT ATC AAG TCC AAT CTT
Glu Ser Gln Gly Glu Val Asp Pro Glu Asp Arg Tyr Val Ile Lys Ser Asn Leu

CAG AAA CTT AAC TGT TGC CTG CCT ACA TCT GCG AAT GAC TCT GCG CTG CCA GGG
Gln Lys Leu Asn Cys Cys Leu Pro Thr Ser Ala Asn Asp Ser Ala Leu Pro Gly

GTC TTC ATT CGA GAT CTG GAT GAC TTT CGG AAG AAA CTG AGA TTC TAC ATG GTC
Val Phe Ile Arg Asp Leu Asp Asp Phe Arg Lys Lys Leu Arg Phe Tyr MET Val

CAC CTT AAC GAT CTG GAG ACA GTG CTA ACC TCT AGA CCA CCT CAG CCC GCA TCT
His Leu Asn Asp Leu Glu Thr Val Leu Thr Ser Arg Pro Pro Gln Pro Ala Ser

GGC TCC GTC TCT CCT AAC CGT GGA ACC GTG GAA TGT TAA AACAGCAGGC AGAGCACCTA
Gly Ser Val Ser Pro Asn Arg Gly Thr Val Glu Cys

AAGTCTGAAT GTTCCTCATG G .

ligasiert, besteht.

**21.** Mäuse-Interleukin-3 wie in Fig. 4 gezeigt, in im wesentlichen reiner Form, wenn es nach einem der Verfahren von einem jeden der Ansprüche 13 bis 17 hergestellt ist.

**Revendications**

**1.** Procédé de clonage moléculaire d'ADNc d'interleukine-3 murine, dans lequel:
(i) on cultive une lignée cellulaire capable de produire de l'interleukine-3 murine dans un milieu de culture;
(ii) on isole l'ARNm produit par les cellules cultivées;
(iii) on fractionne l'ARNm de l'étape (ii) sur un gradient de densité de saccharose et on identifie la fraction ayant l'activité de traduction d'interleukine-3 en utilisant une lignée cellulaire dépendante envers l'interleukine-3;
(iv) on prépare une bibliothèque d'ADNc à partir de l'ARNm de l'étape (iii) en synthétisant un ADNc à deux brins et en insérant l'ADNc résultant dans un vecteur approprié pour générer une banque de molécules d'ADN recombinant, et on transforme la bibliothèque d'ADNc dans une cellule-hôte appropriée;
(v) on rassemble les clones de la bibliothèque d'ADNc transformé, on cultive les masses et on isole l'ADN des clones rassemblés, on transforme l'ADN de plasmide isolé en une forme linéaire avec une endonucléase appropriée, et on enlève ou on détruit tout éventuel ARN contaminant;
(vi) on hybride l'ADN avec l'ARNm isolé à partir d'une lignée cellulaire capable de produire de l'interleukine-3 murine;
(vii) on élue l'ARNm lié et on identifie l'ARNm lié qui a une activité de traduction d'interleukine-3 en utilisant une lignée cellulaire dépendante envers l'interleukine-3, identifiant ainsi le clone Possédant l'ADNc pour l'interleukine-3 murine; et
(viii) on isole et on purifie l'ADNc pour l'interleukine-3 murine à partir du clone identifié dans l'étape (vii).

**2.** ADNc préparé à partir du procédé de la revendication 1.

3. Polynucléotide ayant une séquence équivalant à l'ADNc pour l'interleukine-3 murine ou une de ses parties telle que représentée dans la figure 4 sous forme sensiblement pure.

4. Polynucléotide complémentaire du polynucléotide de la revendication 3.

5. Polynucléotide comprenant un ARN correspondant au polynucléotide de la revendication 3 ou de la revendication 4.

6. Interleukine-3 murine sous forme sensiblement pure et ayant une séquence d'acides aminés telle que représentée dans la figure 4 ou les dessins joints.

7. Echantillon d'hybridation utile pour identifier l'ADN ou l'ARN de l'interleukine-3 dans les cellules eucaryotiques, l'échantillon comprenant un polynucléotide ayant au moins environ dix bases, le polynucléotide ayant une séquence correspondant à l'ADNc de l'interleukine-3 murine, ou son ADN complémentaire, ou son ADN à deux brins, ou un ARN ayant une séquence correspondant à la séquence d'ARNm de l'interleukine-3 murine, et une marque attachée au polynucléotide, ayant une séquence équivalant sensiblement à l'ADNc pour l'interleukine-3 murine ou une de ses parties telle que représentée dans la figure 4 sous forme sensiblement pure.

8. Echantillon de la revendication 7 dans lequel la marque est choisie parmi une marque radioactive ou la biotine.

9. Echantillon de la revendication 7 ou 8 dans lequel le polynucléotide comprend au moins quinze bases.

10. Echantillon de l'une quelconque des revendications 7 à 9 dans lequel le polynucléotide a une séquence correspondant à un ADNc d'interleukine-3 murine ou une de ses parties.

11. Procédé pour identifier l'ADN ou l'ARN d'interleukine-3 dans des cellules eucaryotiques ou des coupes tissulaires ou leurs extraits, caractérisé en ce qu'on utilise l'échantillon de l'une quelconque des revendications 7 à 10.

12. ADNc pour interleukine-3 murine sensiblement tel que décrit ci-dessus et tel que représenté dans la figure 4.

13. Procédé de production d'interleukine-3 murine biologiquement active ou d'une de ses parties ayant une séquence d'acides aminés sensiblement telle que représentée dans la figure 4, ce prodédé comprenant:
(i) l'extraction d'un ADN à haut poids moléculaire à partir d'un tissu murin ou de cellules cultivées, la digestion de l'ADN pour générer des fragments d'ADN, le fractionnement de la taille des fragments d'ADN et le clonage des fragments dans un vecteur de phage approprié, la transfection d'un hôte approprié avec les phages recombinants, l'identification de clones contenant un ADN encodant l'interleukine-3 murine par hybridation avec un échantillon selon la revendication 10, et l'isolement et la purification de l'ADN encodant l'interleukine-3 murine identifié avec l'échantillon;
(ii) l'insertion de l'ADN isolé et purifié dans un vecteur de clonage approprié de manière soit que la région de codage pour l'interleukine-3 murine soit en phase avec une protéine procaryotique, soit que la région de codage pour l'interleukine-3 murine soit sous le contrôle d'un promoteur procaryotique dans le vecteur, pour aboutir à un vecteur de clonage recombinant; et
(iii) la transformation de cellules procaryotiques avec le vecteur de clonage recombinant et la culture des cellules pour produire l'interleukine-3 murine.

14. Procédé de la revendication 13 dans lequel l'interleukine-3 murine est exprimée sous la forme d'une protéine de fusion.

15. Procédé de la revendication 14 dans lequel la protéine de fusion comprend de l'interleukine-3 murine mûre et un oligopeptide comprenant au moins les 9 premiers acides aminés de la -galactosidase.

16. Procédé de la revendication 13 dans lequel l'interleukine-3 murine est exprimée sous la forme d'une protéine de non-fusion.

**17.** Procédé de la revendication 16 dans lequel la protéine de non-fusion commence à l'un quelconque des 32-40 premiers acides aminés de l'interleukine-3 murine.

**18.** Clone recombinant pBTA610 qui se compose de: pUC8 , digéré avec BamHI et d'extrémité remplie avec Klenow dans lequel a été inséré le fragment à 4756pb de Hind III/Nco I

```
                                                            A GCT TCA
                                                              Ala Ser

ATC AGT GGC CGG GAT ACC CAC CGT TTA ACC AGA ACG TTG AAT TGC AGC TCT ATT
Ile Ser Gly Arg Asp Thr His Arg Leu Thr Arg Thr Leu Asn Cys Ser Ser Ile

GTC AAG GAG ATT ATA GGG AAG CTC CCA GAA CCT GAA CTC AAA ACT GAT GAT GAA
Val Lys Glu Ile Ile Gly Lys Leu Pro Glu Pro Glu Leu Lys Thr Asp Asp Glu

GGA CCC TCT CTG AGG AAT AAG AGC TTT CGG AGA GTA AAC CTG TCC AAA TTC GTG
Gly Pro Ser Leu Arg Asn Lys Ser Phe Arg Arg Val Asn Leu Ser Lys Phe Val

GAA AGC CAA GGA GAA GTG GAT CCT GAG GAC AGA TAC GTT ATC AAG TCC AAT CTT
Glu Ser Gln Gly Glu Val Asp Pro Glu Asp Arg Tyr Val Ile Lys Ser Asn Leu

CAG AAA CTT AAC TGT TGC CTG CCT ACA TCT GCG AAT GAC TCT GCG CTG CCA GGG
Gln Lys Leu Asn Cys Cys Leu Pro Thr Ser Ala Asn Asp Ser Ala Leu Pro Gly

GTC TTC ATT CGA GAT CTG GAT GAC TTT CGG AAG AAA CTG AGA TTC TAC ATG GTC
Val Phe Ile Arg Asp Leu Asp Asp Phe Arg Lys Lys Leu Arg Phe Tyr MET Val

CAC CTT AAC GAT CTG GAG ACA GTG CTA ACC TCT AGA CCA CCT CAG CCC GCA TCT
His Leu Asn Asp Leu Glu Thr Val Leu Thr Ser Arg Pro Pro Gln Pro Ala Ser

GGC TCC GTC TCT CCT AAC CGT GGA ACC GTG GAA TGT TAA AACAGCAGGC AGAGCACCTA
Gly Ser Val Ser Pro Asn Arg Gly Thr Val Glu Cys

AAGTCTGAAT GTTCCTCATG GCCCATG
```

d'extrémité remplie avec Klenow.

**19.** Clone recombinant pBTA611 qui se compose de: pUC9, digéré avec Pst I, dans lequel a été inséré le fragment à 450 pb d'HaeIII

```
           C CGG GAT ACC CAC CGT TTA ACC AGA ACG TTG AAT TGC AGC TCT ATT
             Arg Asp Thr His Arg Leu Thr Arg Thr Leu Asn Cys Ser Ser Ile

GTC AAG GAG ATT ATA GGG AAG CTC CCA GAA CCT GAA CTC AAA ACT GAT GAT GAA
Val Lys Glu Ile Ile Gly Lys Leu Pro Glu Pro Glu Leu Lys Thr Asp Asp Glu

GGA CCC TCT CTG AGG AAT AAG AGC TTT CGG AGA GTA AAC CTG TCC AAA TTC GTG
Gly Pro Ser Leu Arg Asn Lys Ser Phe Arg Arg Val Asn Leu Ser Lys Phe Val

GAA AGC CAA GGA GAA GTG GAT CCT GAG GAC AGA TAC GTT ATC AAG TCC AAT CTT
Glu Ser Gln Gly Glu Val Asp Pro Glu Asp Arg Tyr Val Ile Lys Ser Asn Leu

CAG AAA CTT AAC TGT TGC CTG CCT ACA TCT GCG AAT GAC TCT GCG CTG CCA GGG
Gln Lys Leu Asn Cys Cys Leu Pro Thr Ser Ala Asn Asp Ser Ala Leu Pro Gly

GTC TTC ATT CGA GAT CTG GAT GAC TTT CGG AAG AAA CTG AGA TTC TAC ATG GTC
Val Phe Ile Arg Asp Leu Asp Asp Phe Arg Lys Lys Leu Arg Phe Tyr MET Val

CAC CTT AAC GAT CTG GAG ACA GTG CTA ACC TCT AGA CCA CCT CAG CCC GCA TCT
His Leu Asn Asp Leu Glu Thr Val Leu Thr Ser Arg Pro Pro Gln Pro Ala Ser

GGC TCC GTC TCT CCT AAC CGT GGA ACC GTG GAA TGT TAA AACAGCAGGC AGAGCACCTA
Gly Ser Val Ser Pro Asn Arg Gly Thr Val Glu Cys

AAGTCTGAAT GTTCCTCATG G
```

ligaturé à des lieurs Pst I d(pGCTGCAGC) et digéré avec Pst I.

**20.** Clone recombinant pBTA640 constitué de: ptac12, digéré avec PvuII et ligaturé avec le lieur 22mère d-(pAAATGGCTTCAATCAGTGGCCA), digéré avec BalI et traité à la phosphatase et ligaturé avec le fragment à 450pb de HaeIII

C CGG GAT ACC CAC CGT TTA ACC AGA ACG TTG
y Arg Asp Thr His Arg Leu Thr Arg Thr Leu

AAT TGC AGC TCT ATT
Asn Cys Ser Ser Ile

GTC AAG GAG ATT ATA GGG AAG CTC CCA GAA CCT GAA CTC AAA ACT GAT GAT GAA
Val Lys Glu Ile Ile Gly Lys Leu Pro Glu Pro Glu Leu Lys Thr Asp Asp Glu

GGA CCC TCT CTG AGG AAT AAG AGC TTT CGG AGA GTA AAC CTG TCC AAA TTC GTG
Gly Pro Ser Leu Arg Asn Lys Ser Phe Arg Arg Val Asn Leu Ser Lys Phe Val

GAA AGC CAA GGA GAA GTG GAT CCT GAG GAC AGA TAC GTT ATC AAG TCC AAT CTT
Glu Ser Gln Gly Glu Val Asp Pro Glu Asp Arg Tyr Val Ile Lys Ser Asn Leu

CAG AAA CTT AAC TGT TGC CTG CCT ACA TCT GCG AAT GAC TCT GCG CTG CCA GGG
Gln Lys Leu Asn Cys Cys Leu Pro Thr Ser Ala Asn Asp Ser Ala Leu Pro Gly

GTC TTC ATT CGA GAT CTG GAT GAC TTT CGG AAG AAA CTG AGA TTC TAC ATG GTC
Val Phe Ile Arg Asp Leu Asp Asp Phe Arg Lys Lys Leu Arg Phe Tyr MET Val

CAC CTT AAC GAT CTG GAG ACA GTG CTA ACC TCT AGA CCA CCT CAG CCC GCA TCT
His Leu Asn Asp Leu Glu Thr Val Leu Thr Ser Arg Pro Pro Gln Pro Ala Ser

GGC TCC GTC TCT CCT AAC CGT GGA ACC GTG GAA TGT TAA AACAGCAGGC AGAGCACCTA
Gly Ser Val Ser Pro Asn Arg Gly Thr Val Glu Cys

AAGTCTGAAT GTTCCTCATG G .

**21.** Interleukine-3 murine telle que représentée dans la figure 4 sous forme sensiblement pure lorsqu'elle est produite par le procédé de l'une quelconque des revendications 13 à 17.

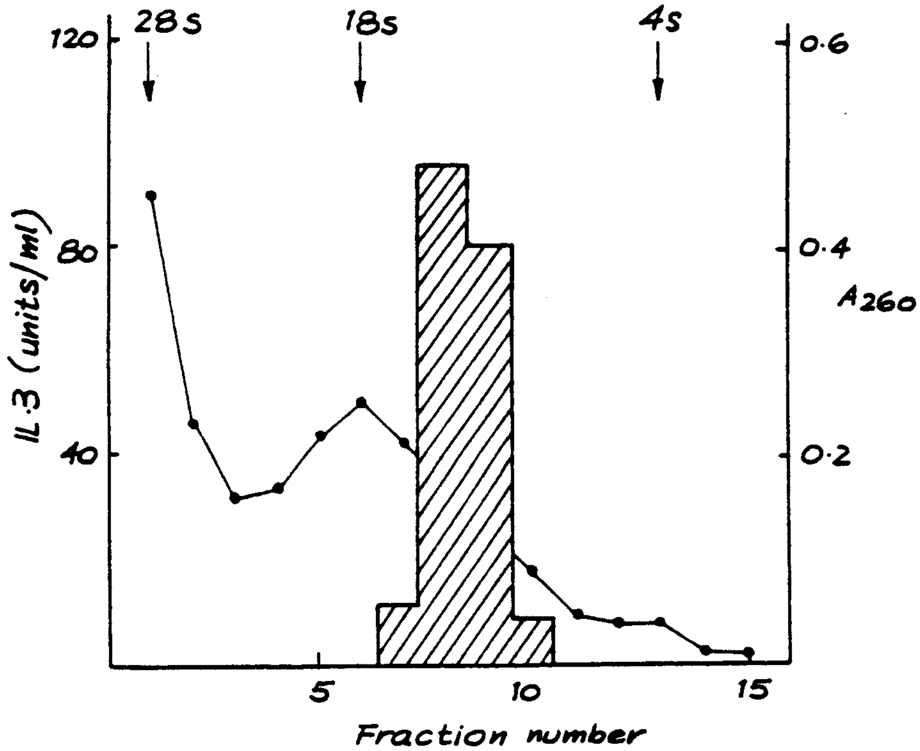
120
80
40
28S
18S
4S
0.6
0.4
0.2
A260
IL.3 (units/ml)
5
10
15
Fraction number

FIG.1

FIG.3

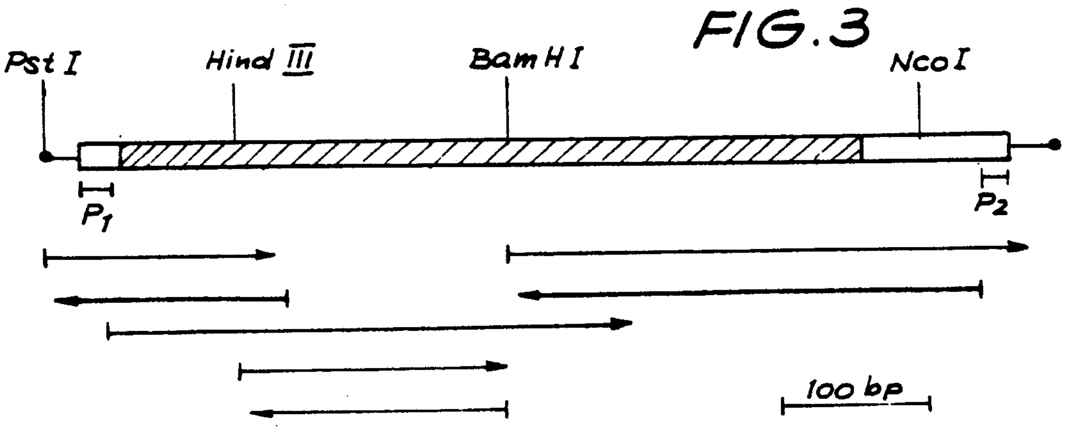
Pst I
Hind III
Bam H I
Nco I
P1
P2
100 bp

FIG. 2

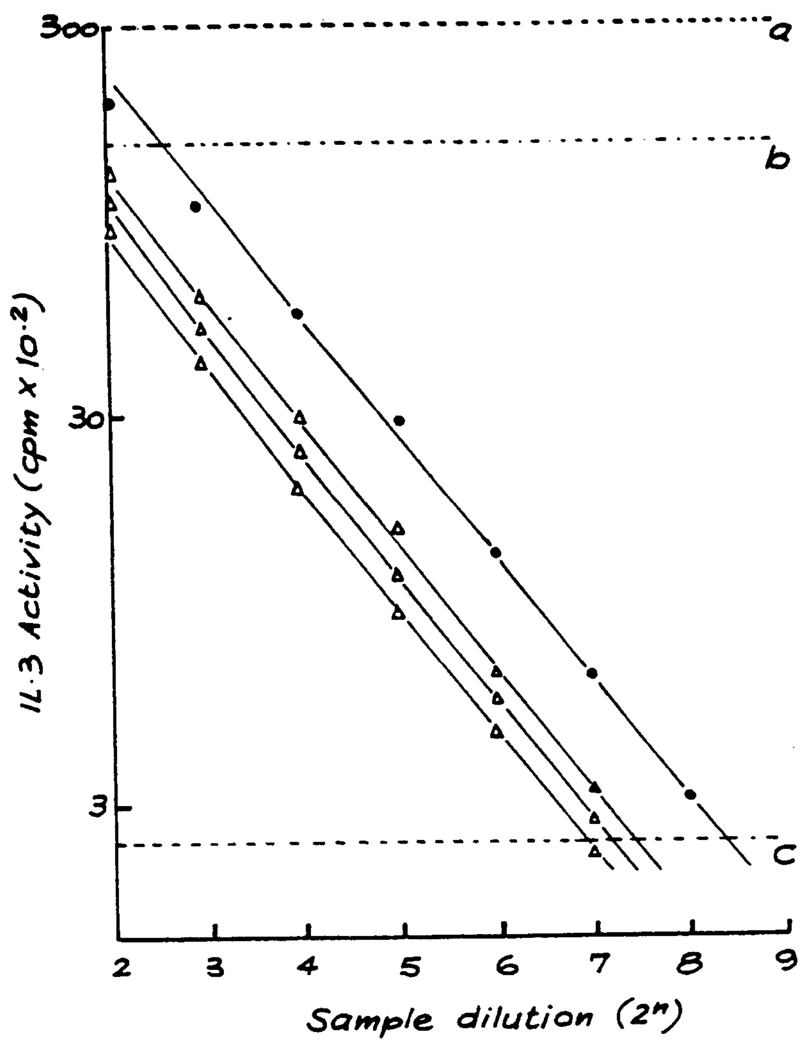


Fig. 2 Titration curves for the IL-3 activity present in oocyte translates. Δ, Translates of mRNA hybridizing to PILM1 (see Table 2); ●, translates of WEH1-3mRNA. Twofold dilutions of the oocyte translates were made in microtitre trays and the IL-3 activity present was measured using the IL-3 dependent cell line 32Dcl-23 as described previously. The stimulation of growth of the indicator cells is expressed in terms of $^{3}$H-thymidine incorporation. The dotted lines designated a, b and c represent incorporations of 100%, 50% and background plus three standard deviations respectively.

```
                              M  V  L  A  S  S  T  T  S  I  H  T  M  L  L  L  L  L  M  L  F
AACCCCTTGGAGGACCAGAACGAGACAATGGTTCTTGCCAGCTCTACCACCAGCATCCACACCATGCTGCTCCTGCTCCTGATGCTCTTC
        10        20        30        40        50        60        70        80        90
                                    D  T  H  R  L  T  R  T  L  X  X  S  S  I  V
 H  L  G  L  Q  A  S  I  S  G  R  D  T  H  R  L  T  R  T  L  N  C  S  S  I  V  K  E  I  I
CACCTGGGACTCCAAGCTTCAATCAGTGGCCGGGATACCCACCGTTTAACCAGAACGTTGAATTGCAGCTCTATTGTCAAGGAGATTATA
       100       110       120       130       140       150       160       170       180
 G  K  L  P  E  P  E  L  K  T  D  D  E  G  P  S  L  R  N  K  S  F  R  R  V  N  L  S  K  F
GGGAAGCTCCCAGAACCTGAACTCAAAACTGATGATGAAGGACCCTCTCTGAGGAATAAGAGCTTTCGGAGAGTAAACCTGTCCAAATTC
       190       200       210       220       230       240       250       260       270
 V  E  S  Q  G  E  V  D  P  E  D  R  Y  V  I  K  S  N  L  Q  K  L  N  C  C  L  P  T  S  A
GTGGAAAGCCAAGGAGAAGTGGATCCTGAGGACAGATACGTTATCAAGTCCAATCTTCAGAAACTTAACTGTTGCCTGCCTACATCTGCG
       280       290       300       310       320       330       340       350       360
 N  D  S  A  L  P  G  V  F  I  R  D  L  D  D  F  R  K  K  L  R  F  Y  M  V  H  L  N  D  L
AATGACTCTGCGCTGCCAGGGGTCTTCATTCGAGATCTGGATGACTTTCGGAAGAAACTGAGATTCTACATGGTCCACCTTAACGATCTG
       370       380       390       400       410       420       430       440       450
 E  T  V  L  T  S  R  P  P  Q  P  A  S  G  S  V  S  P  N  R  G  T  V  E  C
GAGACAGTGCTAACCTCTAGACCACCTCAGCCCGCATCTGGCTCCGTCTCTCCTAACCGTGGAACCGTGGAATGTTAAAACAGCAGGCAG
       460       470       480       490       500       510       520       530       540

AGCACCTAAAGTCTGAATGTTCCTCATGGCCCATGGTCAAAAGGATTTTACATTCCTTTATGCCATCAAATGTCTTATCAATTTATCTA
       550       560       570       580       590       600       610       620
```

FIGURE 4

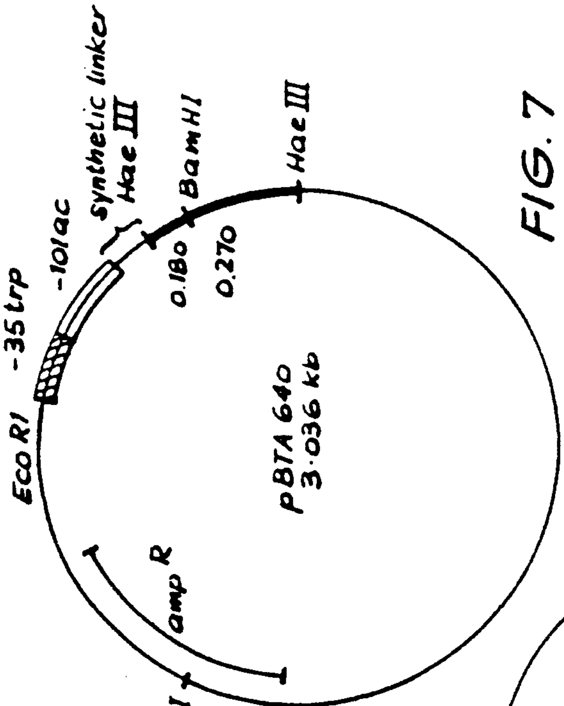


FIG. 7

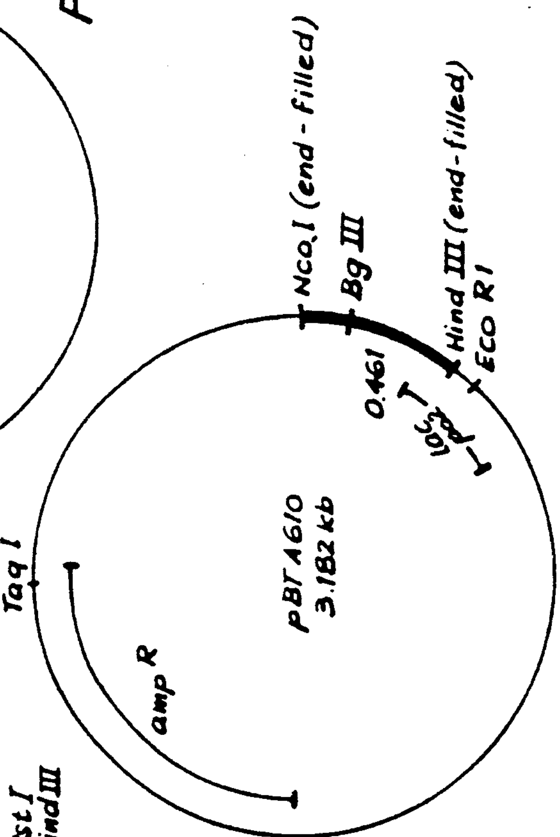


FIG. 6

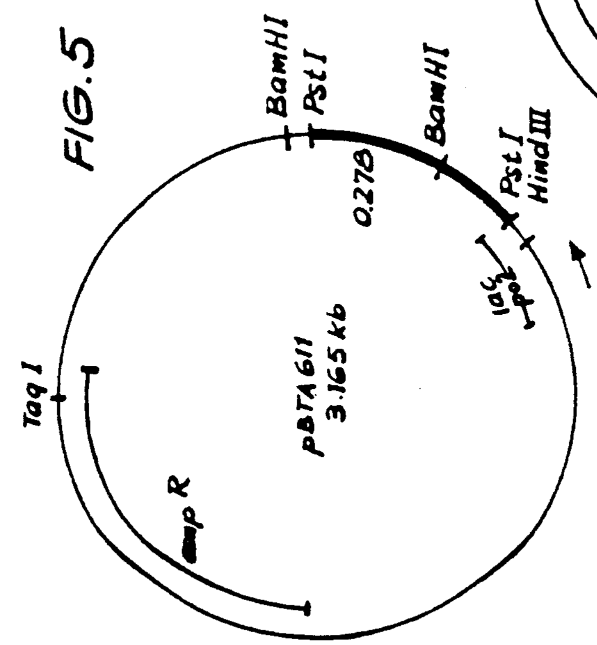


FIG. 5

```
                 ┌──────── B-gal ─────────────────┐ ┌─IL-3 ────────
                  1   2   3   4   5   6   7   8   9   10  32  33  34  35
                 Thr Met Ile Thr Pro Ser Leu Ala Ala Ala Arg Asp Thr His
BTA611: ATG ACC ATG ATT ACG CCA AGC TTG GCT GCA GCC CGG GAT ACC CAC ....

                 ┌──────── B-gal ──────────────┐ ┌──── IL-3 ─────────────────────
                  1   2   3   4   5   6   7   8   9   27  28  29  30  31  32  33  34  35
                 Thr Met Ile Thr Asn Ser Arg Gly Ser Ala Ser Ile Ser Gly Arg Asp Thr His
BTA610: ATG ACC ATG ATT ACG AAT TCC CGG GGA TCA GCT TCA ATC AGT GGC CGG GAT ACC CAC....

                 ┌──────── IL-3 ──────────────
                  27  28  29  30  31  32  33  34  35
                 Ala Ser Ile Ser Gly Arg Asp Thr His
BTA640: ATG GCT TCA ATC AGT GGC CGG GAT ACC CAC......
```

FIG 8.

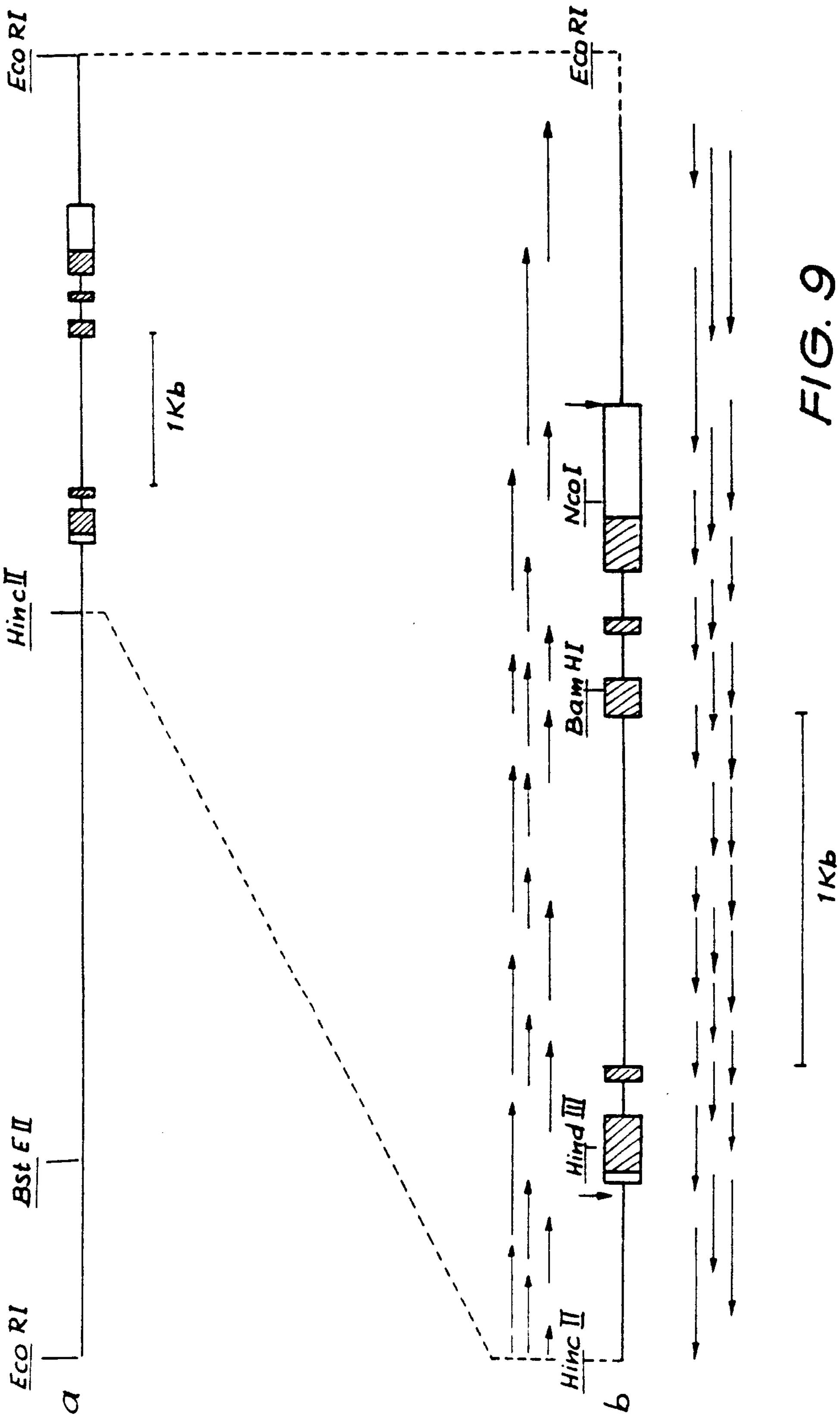
a
EcoRI
BstEII
HincII
EcoRI
1Kb
b
HincII
HindIII
BamHI
NcoI
EcoRI
1Kb

FIG. 9

FIG. 10

```
CTAGAGATACACTTAGCTCTGGCTACCACCAGCCAGACGACTAGCCCACACATGGAAGTTTAACCATGTGCCAGAATGCCTACCAAGTTCAAGTCTGCTCCAGTGACTCTGGTGACTACC  120
TCTATCAGGTAGGGTGTACAGTCACCACATAGGCTGTACAGTCAGGGTCAAGTTTGTGCAAGGGATGGTAGGATGAGATTCCACTGCATAGAAAGCCCAAGCTGGCTCAGAGCCAGGCTA  240
CTTCCTCCCACAACCTGTTTCCACTCCGTCCATCTCTATGACAAAGGAAGAAGATGGCCTTTGAATAAGCAGTCTTTCTTCCCATGTCGATAATCTTGAGTACTAGAAAGTGATGAATAA  360
GTTTGTGGTTTGCTATGGAGGTTCCATGTCAGATAAAGCTGCTTCTGATGCCTGCCCTCCCCCCTGCCCCGCCGGGCCCCGCCCCACCCCTCTCTGAATACATATAAGGTGAAGGCTCCT  480
                                                                    10                  20
                                       M  V  L  A  S  S  T  T  S  I  H  T  M  L  L  L  L  L  M  L  F  H  L  G  L  Q  A
GTGGCTTCTTCAGAACCCCTTGGAGGACCAGAACGAGACAATGGTTCTTGCCAGCTCTACCACCAGCATCCACACCATGCTGCTCCTGCTCCTGATGCTCTTCCACCTGGGACTCCAAGC  600
      30                  40                  50
 S  I  S  G  R  D  T  H  R  L  T  R  T  L  N  C  S  S  I  V  K  E  I  I  G  K  L  P ┌Intron 1 ──►
TTCAATCAGTGGCCGGGATACCCACCGTTTAACCAGAACGTTGAATTGCAGCTCTATTGTCAAGGAGATTATAGGGAAGCTCCCAGTGAGTAACTGGCTGAGGTTGGCCTGGTGCAGGCC  720
                                                   60
                                  ◄── Intron 1 ┐ E  P  E  L  K  T  D  D  E  G  P  S  L  R ┌Intron 2 ──►
GGCTCCAACAGGTGCCTCAAGCCAGTGAGCCTCATGATTCTTTCTTTCTATGTCCTCACAGGAACCTGAACTCAAAACTGATGATGAAGGACCCTCTCTGAGGGTAAGAGCCCTGCTTTG  840
GGATATTCTTGGGTTCCATCTATCTCCTGCCTGGGTGACTTCAGCCATCACTCCACGATGCCTTGCTTCCATTTTTGCATCTATCTCAGTGGGGTTATTAAGGAAATCATCAGATGACTC  960
TCAAGCCTCAGTATATACCTCAGTCAGCTGCAATAATGAAAGTTACCTTTTAGGATATACAATGAAGACAGCTGTGGGTGAACCCTGCCTGCTGCAGGCCTCTGGCTCCACTTTCAGTGG  1080
GGATGCCATTGCCCTCTGTGACTTTTGTGTCTTTTGCTTTTCTTCCTCCTCCAAAACTGAAGTTTGTGTTTTCTACTTCCGCCAGCCCCAGACATTACTATTTGTAGTTATTTTCCTAGT  1200
TTGATACAATAGTTATGTCTTGTTTTTATTTGTTTGGACCTAACATGAAGTTCTTTGCAAGAGTGCTGAATGTCTATATTTCCCCTCCCTAGGAACATGTTGGAGCCTATAGGATTTTCT  1320
TCTAGGTATCGAGAAATTCTTAATTAAATTAAAGCATTGGCCTGGGTTTTTGGCATCTTGGTATTTTCCTTGGCCAACCTTCTGCCTTTTCTAGAGCTTTTCTGGAGGGATGTGTTTTCC  1440
TCAAGTACAGACAAATCTGCTTAGATCCTTCACACAGCTCACAGGCTGCCAGGAGTTAAGACCTGGTGCTTGGGAGAAACAGGCCCTTGTCTGAGATATACACTAGCTTTTAGCCCCAGG  1560
ATAATGAAGGGACAGGAATAAGGCTGTTCAAAGAAATCTCAAATAGCAGGCACACTTCCCTAGCTCTGATCTCTCCAGCTCTCACTTCCCAACTCTCACCTCCCCAGCTCTTTCCTTTCC  1680
AGTTCTCACCTCCCTGGCTCTCACCTCTCTGGATCTCATCTCCCTGGCTCTCAACTCCCAAGCTCTTTCATTTCCAGTTCTCACCTCCCAGCTCTCACATCCCTGGGCCTTACATAAAGA  1800
          70                  80                  90                  100
◄── Intron 2 ┐ N  K  S  F  R  R  V  N  L  S  K  F  V  E  S  Q  G  E  V  D  P  E  D  R  Y  V  I  K  S  N  L  Q |Intron 3►
TTTCTTGCCCTCTTAGAATAAGAGCTTTCGGAGAGTAAACCTGTCCAAATTCGTGGAAAGCCAAGGAGAAGTGGATCCTGAGGACAGATACGTTATCAAGTCCAATCTTCAGGTGTGTGG  1920
AGCTGCAGAGGGTTGGGGGTGGGGGGTGGGGCTCCGTGCCTTTACCTTTCCCAGCATTTTTGGAGAGGAAAGTAGGGCCTTCTCCACTTTAGGAGGGATCTGACTATGCCTTGGTGTCTC  2040
                      110
◄─────┐ K  L  N  C  C  L  P  T  S  A  N  D  S  A ┌Intron 4 ──►
TCCACAGAAACTTAACTGTTGCCTGCCTACATCTGCGAATGACTCTGCGGTAAGCTGCCCTTCCCCCACGTGTTTAGCAGGCTGTGCCCTTGCCCATGACCAGACTTATAGTTTGCTGTG  2160
                                                              120                 130
                                  ◄── Intron 4 ┐ L  P  G  V  F  I  R  D  L  D  D  F  R  K  K  L  R  F  Y  M  V  H  L
GCCCGCAGCAACATGTTCACATCTAATGCCTTCTTTTCTGGTTCCTCACAGCTGCCAGGGGTCTTCATTCGAGATCTGGATGACTTTCGGAAGAAACTGAGATTCTACATGGTCCACCTT  2280
  140                  150                  160
 N  D  L  E  T  V  L  T  S  R  P  P  Q  P  A  S  G  S  V  S  P  N  R  G  T  V  E  C  *
AACGATCTGGAGACAGTGCTAACCTCTAGACCACCTCAGCCCGCATCTGGCTCCGTCTCTCCTAACCGTGGAACCGTGGAATGTTAAAACAGCAGGCAGAGCACCTAAAGTCTGAATGTT  2400
CCTCATGGCCCATGGTCAAAAGGATTTTACATTCCTTTATGCCATCAAATGTCTTATCAATTTATCTACTTTCTGAAATTTACAACTCTCCTTTGGCTTTACCTAATTATGTTCCTATTT  2520
TATTCCATTAAGGCTATTTATTTATGTATTTATGTATTTATTTATTTATTGCCTTCTGTGATGTGAGTATATCTGTTTTAGCTGAGGAGGAGTTTCTCCAAAGAAAATTCCAAGGAAGAC  2640
TGGGGCCATGTTCATTTGTCCCTTGTGGAAATAAATAACTTTGAACAAATGGGTTTGGTTTCGTCCAGTTTCTTGAGGAGTTTGGATTGATCTCTGTAACAGTCCTAGAGGATACTTGTC  2760
CGGACATCACATGCGGCTACACAGCATGCAACATGTATCAGATCTTGGTCTGGACATTTAACCTTTACTATTTAATCTACTCTTCACTCCAAACCAGAATGTGTGATCTCTGTTTTATGG  2880
GACTACAGAAGTCCAGTTTGGCAAAGTTAGACCAAGTAAATGCAGCTTCTAACTAGTGCCCCCCTCTAGGACTTCATTCAGGCTTGGCGCCTGAGAACATGCTGAAATCCATTAGAATGC  3000
CTCCCCTAGTGGTCATACTTAGAACTGCAGCAGTGTCCCTAACACTTCCTGTAGTGGAAATCTATCCACTACCACCATCATCTGAGCTCCCAACTAACTGGCAGGACTCTCTGAGGGTGC  3120
AGGTTACTCAGACAAAGAAATCTCACTTTTTCCAGAAGTTTCCATGTCTGCCTAGGCTTCTGGCTTCTCTTGGTTGATTTATTCTCCCTACCTCGTGCCTGACTCCAGACTGACCTTGGG  3240
ATTCATGACTTATTGAGCCGGATTGAGCTTTAAAAAGGCGTCTTTGAGCCTTAACCTTAAATCGTTAGAGGACTTACCTATGGGCTTGCCTGTATCCTAAGGCTGTCATATGGGACGCGC  3360
CCTCACCTTTTGCTTGTGTTTCCTCACGGTTATCAACGCTGTGGGAGCTCCAGCAAACAAAAGTGCTGTGGCTTTCCAGACCTTCCCCCTCCCTTTCTTTCTACCTAAACTTTCAGCCAC  3480
CTTCAGCTCA
```